(19) Europäisches Patentamt
European Patent Office
Office européen des brevets

(11) Veröffentlichungsnummer : **0 013 960**
**B1**

(12) # EUROPÄISCHE PATENTSCHRIFT

(45) Veröffentlichungstag der Patentschrift : **03.11.82**

(21) Anmeldenummer : **80100273.4**

(22) Anmeldetag : **21.01.80**

(51) Int. Cl.³ : **C 07 C 49/84**, C 07 C 45/74, C 07 C 69/017, C 07 C 69/96, C 07 C 97/10, C 07 C101/19, C 07 C121/76, C 07 C149/32, C 07 D207/333, C 07 D213/50, C 07 D213/80// C07D241/24, C07D307/46, C07D317/54, C07D333/22

(54) **Substituierte Acetophenone, diese enthaltende Präparate und Verfahren zu deren Herstellung.**

(30) Priorität : **26.01.79 GB 7902907**

(43) Veröffentlichungstag der Anmeldung : **06.08.80 (Patentblatt 80/16)**

(45) Bekanntmachung des Hinweises auf die Patenterteilung : **03.11.82 Patentblatt 82/44**

(84) Benannte Vertragsstaaten : **AT BE CH DE FR GB IT LU NL SE**

(73) Patentinhaber : **F. HOFFMANN-LA ROCHE & CO. Aktiengesellschaft**
**CH-4002 Basel (CH)**

(72) Erfinder : **Fujiu, Morio**
**Zengyo Danchi 5-7-203 Fujisawa 3768-3**
**Fujisawa-shi, Kanagawaken (JP)**
Erfinder : **Suhara, Yasuji**
**Dream Height 9-506 Matano-cho 1403 banchi**
**Totsuka-ku, Yokohama-shi Kanagawa-ken (JP)**
Erfinder : **Ishitsuka, Hideo**
**1-go 405, Katsura-cho 1-banchi 1**
**Totsuka-ku Yokohama-shi, Kanagawa-ken (JP)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

(56) Entgegenhaltungen :

**HOUBEN-WEYL « Methoden der organischen Chemie », 4. Auflage, Band 7, Teil 2b, 1976, GEORG THIEME VERLAG, Stuttgart. Seiten 1 482-1 491**

**H. BEYER « Lehrbuch der organischen Chemie », 17. Auflage, 1973, S. HIRZEL VERLAG, Stuttgart. Seite 470**

**BEILSTEINS Handbuch der organischen Chemie, 4. Auflage, 8. Band, 1925, JULIUS SPRINGER, Berlin. Seiten 432, 433, 501-503, 543, 545**

**BEILSTEINS Handbuch der organischen Chemie, 4. Auflage, 1. Ergänzungswerk, 7. und 8. Band, 1931, JULIUS SPRINGER, Berlin. Seiten 738, 739**

**BEILSTEINS Handbuch der organischen Chemie, 4. Auflage, 2. Ergänzungswerk, 8. Band, 1948, SPRINGER-VERLAG, Berlin-Göttingen-Heidelberg. Seiten 378, 481, 479**

**BEILSTEINS Handbuch der organischen Chemie, 4. Auflage, 3. Ergänzungswerk, 8. Band, Teil 3, 1970, SPRINGER-VERLAG, Berlin-Heidelberg-New-York. Seite 2 833**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 013 960 B1**

Imprimerie Jouve, 18, rue St-Denis, 75001 Paris, France

BEILSTEINS Handbuch der organischen Chemie, 4. Auflage, 3. Ergänzungswerk, 8. Band, Teil 4, 1970, SPRINGER-VERLAG, Berlin-Heidelberg-New-York. Seiten 3 713, 4 114, 4 115, 4 261

Substituierte Acetophenone, diese enthaltende Präparate und Verfahren zu deren Herstellung

Die vorliegende Erfindung betrifft neue substituierte Acetophenone, ein Verfahren zu deren Herstellung und antivirale Mittel auf der Basis der neuen und bekannter Acetophenone.

Die vorliegende Erfindung betrifft insbesondere antivirale Mittel enthaltend ein substituiertes Acetophenon der Formel

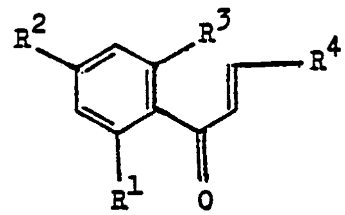

(I)

worin

$R^1$ Hydroxy, ein von einer aliphatischen Mono- oder Dicarbonsäure mit 2-18 C-Atomen oder einer heterocyclischen Carbonsäure die Stickstoff enthält, abgeleitetes Acyloxy, oder Aminoacyloxy ;

$R^2$ niederes Alkoxy,

$R^3$ Wasserstoff oder niederes Alkoxy ; und

$R^4$ einen Phenylrest, der durch einen oder mehrere Substituenten aus der Gruppe niederes Alkyl, niederes Alkoxy, Benzyloxy, Allyloxy, Alkylthio, Dialkylamino, Amino, Cyano, Hydroxy, Halogen und Alkylendioxy substituiert sein kann, einen gegebenenfalls durch niederes Alkyl substituierten Pyridyl-, Furyl-, Thienyl oder Pyrrolyl darstellt.

Darüber hinaus betrifft die vorliegende Erfindung die neuen substituierten Acetophenone der allgemeinen Formel

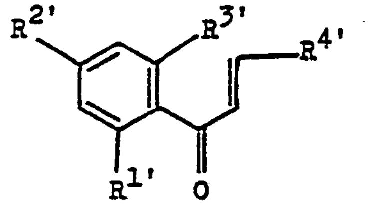

(II)

worin

$R^{1'}$ Hydroxy, ein von einer aliphatischen Mono- oder Di-carbonsäure mit 2-18 C-Atomen oder einer heterocyclischen Carbonsäure, die Stickstoff enthält, abgeleitetes Acyloxy ; oder Aminoacyloxy ;

$R^{2'}$ niederes Alkoxy ;

$R^{3'}$ niederes Alkoxy ; und

$R^{4'}$ einen Phenylrest, der durch einen oder mehrere Substituenten aus der Gruppe niederes Alkyl, niederes Alkoxy, Allyloxy, Alkylthio, Dialkylamino, Amino und Cyano substituiert sein kann, durch niederes Alkyl substituiertes Furyl oder gegebenenfalls durch niederes Alkyl substituiertes Pyridyl, Thienyl oder Pyrrolyl darstellen, mit den Einschränkungen, dass, wenn $R^{4'}$ p-Methoxyphenyl ist, $R^{1'}$ keinen Acetoxyrest darstellt, und dass $R^{2'}$ und $R^{3'}$ nicht gleichzeitig Methoxy oder Aethoxy sind, wenn $R^{1'}$ Hydroxy und $R^{4'}$ Methoxyphenyl oder Dimethoxyphenyl darstellen, Verbindungen, die als Wirksubstanzen antiviraler Mittel verwendet werden können.

Besonders bevorzugte Acyloxygruppen, die sich von einer aliphatischen Mono- oder Dicarbonsäure mit 2-18 Kohlenstoffatomen oder einer heterocyclischen Carbonsäure, die Stickstoff enthält, ableiten, sind Acetoxy, Propionyloxy, Butyryloxy, Isobutyryloxy, Pivaoyloxy, Steraoyloxy, Nicotinoyloxy und Pyrazinylcarbonyloxy. Andere Beispiele solcher Acylreste sind Alkoxycarbonyloxygruppen wie Ethoxycarbonyloxy und Carboxyalkanoyloxygruppen wie 4-Carboxybutanoyloxy. Eine Aminoacyloxygruppe kann sich von einer aliphatischen Aminosäure ableiten, wobei L-Lysyloxy, L-Alanyloxy und L-Glutaminyloxy besonders bevorzugte Gruppen sind. Niedere Alkoxygruppen können 1-4 Kohlenstoffatome enthalten, wie beispielsweise Methoxy, Ethoxy, Propoxy und Isopropoxy. Bevorzugte substituierte Phenylreste sind p-Tolyl, p-Methoxyphenyl, p-Ethoxyphenyl, p-Propoxyphenyl, p-(Benzyloxy)-m-methoxyphenyl, p-(Allyloxy)-phenyl, p-(Methylthio)-phenyl, p-(Dimethylamino)-phenyl, p-(Diethylamino)-phenyl, p-Aminophenyl, p-Cyanophenyl, p-Hydroxyphenyl, p-Hydroxy-m-methoxyphenyl, m,p-(Methylendioxy)-phenyl und p-Chlorphenyl. Bevorzugte Beispiele von Pyridyl-, Furyl-, Thienyl- und Pyrrolylgruppen, die durch niederes Alkyl substituiert sein können, sind 3-Pyridyl, 2-Furyl, 5-Methyl-2-furyl, 2-Thienyl, 3-Methyl-2-thienyl und 1-Methyl-pyrrol-2-yl.

Beispiele für Verbindungen der Formel I sind :

A) Neue Verbindungen (Chalkon = Benzyliden-acetophenon)

4-(Benzyloxy)-2'-hydroxy-3,4',6'-trimethoxychalkon ;
4-Ethoxy-2'-hydroxy-4',6'-dimethoxychalkon ;
2'-Hydroxy-4',6'-dimethoxy-4-propoxychalkon ;
2'-Hydroxy-4',6'-dimethoxy-4-methylchalkon ;
2'-Hydroxy-4',6'-dimethoxy-3-(3-pyridyl)-acrylophenon ;
2'-Hydroxy-4',6'-dimethoxy-4-(methylthio)-chalkon ;
4-(Allyloxy)-2'-hydroxy-4',6'-dimethoxychalkon ;
4-(Dimethylamino)-2'-hydroxy-4',6'-dimethoxychalkon.
4-(Diethylamino)-2'-hydroxy-4',6'-dimethoxychalkon ;
2',4,4'-Triethoxy-6'-hydroxychalkon ;
2'-Hydroxy-4,4'-dimethoxy-6'-propoxychalkon ;
2'-Hydroxy-6'-isopropoxy-4,4'-dimethoxychalkon ;
2'-Ethoxy-6'-hydroxy-4,4'-dimethoxychalkon ;
2'-Hydroxy-4',6'-dimethoxy-3-(2-thienyl)-acrylophenon ;
2'-Hydroxy-4',6'-dimethoxy-3-(3-methyl-2-thienyl)-acrylophenon ;
2'-Hydroxy-4',6'-dimethoxy-3-(5-methyl-2-furyl)-acrylophenon ;
2'-Hydroxy-4',6'-dimethoxy-3-(N-methyl-2-pyrrolyl)-acrylophenon ;
4-Amino-2'-hydroxy-4',6'-dimethoxychalkon ;
4-Cyano-2'-hydroxy-4',6'-dimethoxychalkon ;
2',4,4'-Trimethoxy-6'-(propionyloxy)-chalkon ;
2',4,4'-Trimethoxy-6'-(octadecanoyloxy)-chalkon ;
2'-(Ethoxycarbonyloxy)-4,4',6'-trimethoxychalkon ;
2',4,4'-Trimethoxy-6'-(nicotinoyloxy)-chalkon ;
2',4,4'-Trimethoxy-6'-(pyrazinylcarbonyloxy)-chalkon ;
2'-(L-Alanyloxy)-4,4',6'-trimethoxychalkon ;
2'-(4-Carboxybutanoyloxy)-4,4',6'-trimethoxychalkon ;
4-Ethoxy-2',4'-dimethoxy-6'-(propionyloxy)-chalkon ;
2',4'-Dimethoxy-6'-(propionyloxy)-4-propoxychalkon ;
2',4'-Dimethoxy-4-methyl-6'-(propionyloxy)-chalkon ;
2',4'-Dimethoxy-4-(methylthio)-6'-(propionyloxy)-chalkon ;
4-(Allyloxy)-2',4'-dimethoxy-6'-(propionyloxy)-chalkon ;
2',4,4'-Triethoxy-6'-(propionyloxy)-chalkon ;
2',4'-Dimethoxy-6'-(propionyloxy)-chalkon ;
4-Chloro-2',4'-dimethoxy-6'-(propionyloxy)-chalkon ;
2'-Ethoxy-4,4'-dimethoxy-6'-(propionyloxy)-chalkon ;
2',4'-Dimethoxy-6'-(propionyloxy)-3-(2-thienyl)-acrylophenon ;
2',4'-Dimethoxy-3-(5-methyl-2-furyl)-6'-(propionyloxy)-acrylophenon ;
2',4'-Dimethoxy-3-(1-methylpyrrol-2-yl)-6'-(propionyloxy)-acrylophenon ;
3-(2-Furyl)-2',4'-dimethoxy-6'-(propionyloxy)-acrylophenon ;
2',4'-Dimethoxy-3,4-(methylendioxy)-6'-(propionyloxy)-chalkon ;
4,4'-Dimethoxy-2'-(propionyloxy)-6'-propoxychalkon ;
2'-Isopropoxy-4,4'-dimethoxy-6'-(propionyloxy)-chalkon ;
4'-Ethoxy-2'-hydroxy-4,6'-dimethoxychalkon ;
4,4'-Diethoxy-2'-hydroxy-6'-methoxychalkon ;
2'-Hydroxy-3,4',6'-trimethoxychalkon ;
2',4-Diethoxy-6'-hydroxy-4'-methoxychalkon ;
2'-Hydroxy-3,4',5,6'-tetramethoxychalkon ;
2,2'-Dihydroxy-3,4',6'-trimethoxychalkon ;
4'-Ethoxy-2'-hydroxy-6'-methoxy-3-(5-methyl-2-furyl)-acrylophenon ;
2'-Ethoxy-6'-hydroxy-4'-methoxy-3-(5-methyl-2-furyl)-acrylophenon ;
2',4'-Diethoxy-6'-hydroxy-3-(5-methyl-2-furyl)-acrylophenon ;
2',4,4'-Trimethoxy-6'(pivaloyloxy)-chalkon.

B) Bekannte Verbindungen

2'-Hydroxy-4,4',6'-trimethoxychalkon ;
2',4-Dihydroxy-4',6'-dimethoxychalkon ;
2',4-Dihydroxy-3,4',6'-trimethoxychalkon ;
2'-Hydroxy-4',6'-dimethoxychalkon ;
2'-Hydroxy-4',6'-dimethoxy-3,4-(methylendioxy)-chalkon ;
4-Chloro-2'-hydroxy-4',6'-dimethoxychalkon ;
2'-Hydroxy-4,4'-dimethoxychalkon ;
3-(2-Furyl)-2'-hydroxy-4',6'-dimethoxyacrylophenon ;

2'-Acetoxy-4,4',6'-trimethoxychalkon ;
2',4'-Diethoxy-6'-hydroxy-4-methoxychalkon ;
2',3-Dihydroxy-4,4',6'-trimethoxychalkon ;
2'-Hydroxy-3,4,4',6'-tetramethoxychalkon ;
2'-Hydroxy-2,4,4',6'-tetramethoxychalkon ;
2'-Hydroxy-2,3,4',6'-tetramethoxychalkon.

Von den bekannten Verbindungen die z.B. in Beilstein, 4. Auflage, Hauptwerk 8. Band, Seiten 432, 433, 501-503, 543, 545 ; 1. Erg. S. 738, 739 ; 2. Erg. S. 378, 481, 579 ; 3. Erg. S. 2 833, 3 713, 4 114, 4 115, 4 261 erwähnt sind, ist der Anmelderin eine frühere medizinische Anwendung nicht bekannt.

Erfindungsgemäss können die neuen substituierten Acetophenone der Formel II dadurch hergestellt werden, dass man

a) zwecks Herstellung von Verbindungen der Formel II mit $R^{1'}$ = Hydroxy eine Verbindung der Formel

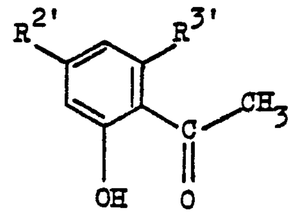

(III)

worin

$R^{2'}$ und $R^{3'}$ wie in Formel II definiert sind, mit einem Aldehyd der Formel

$$R^{4'}—CHO \qquad (IV)$$

worin

$R^{4'}$ wie in Formel II definiert ist, in einem organischen Lösungsmittel in Gegenwart eines basischen Katalysators umsetzt, oder

b) zwecks Herstellung von Verbindungen der Formel II in denen $R^{1'}$ einen wie oben definierten Acyloxyrest oder Aminoacyloxyrest darstellt, eine Verbindung der Formel

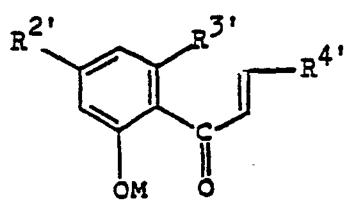

(V)

worin

$R^{2'}$, $R^{3'}$ und $R^{4'}$ wie in Formel II definiert sind und M ein Wasserstoffatom oder ein Alkalimetall bedeutet, mit einem Säureanhydrid oder einem Säurehalogenid, das sich von einer aliphatischen Mono- oder Dicarbonsäure mit 2-18 C-Atomen, einer heterocyclischen Carbonsäure die Stickstoff enthält, oder einer Aminosäure ableitet, in einem organischen Lösungsmittel umsetzt.

Die Verfahrensvariante a) kann so durchgeführt werden, dass man einen basischen Katalysator, z.B. ein Alkalimetallhydroxid oder -carbonat wie Natriumhydroxid, Kaliumhydroxid, Natriumcarbonat oder Kaliumcarbonat, ein Alkoholat, wie Natriumethoxid oder Kaliumethoxid, zu einem Gemisch der Verbindungen der Formeln III und IV in einem organischen Lösungsmittel, z.B. Methanol, Ethanol, Dioxan, Tetrahydrofuran, Benzol oder Hexan, gibt und das Gemisch während einer Zeit von einigen Stunden bis zu 5 Tagen bei einer Temperatur von 0-100 °C rührt. Die gewünschte Verbindung der Formel II kann aus dem Reaktionsgemisch in an sich bekannter Weise isoliert und gereinigt werden, beispielsweise durch Umkristallisation oder Chromatographie. Enthält die Verbindung IV eine Aminogruppe, so sollte diese vor der Umsetzung durch Ueberführung in eine geschützte Aminogruppe, beispielsweise Acetamido, geschützt werden.

Die Umsetzung gemäss Verfahrensvariante b) kann in einem organischen Lösungsmittel, wie Pyridin, Dimethylaminopyridin, Lutidin oder Triethylamin, bei einer Temperatur von 0-80 °C während 1-10 Stunden durchgeführt werden. Bevorzugte Beispiele von Säureanhydriden oder Säurehalogeniden sind Stearinsäureanhydrid, Glutarsäureanhydrid ; Acetylchlorid, Propionylchlorid, Ethylchlorformiat, Nicotinoylchlorid, Pyrazinylcarbonylchlorid und Aminosäurehalogenide. In die Reaktion kann eine aliphatische Mono- oder Dicarbonsäure mit 2-18 Kohlenstoffatomen, eine heterocyclische Carbonsäure, die Stickstoff enthält, oder eine Aminosäure in freier Form zusammen mit einem Reagens, das diese Säure in das entsprechende Halogenid überführt, eingesetzt werden. Bevorzugte Reagenzien sind

Thionylchlorid und Thionylbromid. Wird eine Aminosäure oder deren Halogenid verwendet, so sollte vor der Umsetzung die Aminogruppe in an sich bekannter Weise geschützt werden, beispielsweise durch Ueberführung in eine Benzyloxycarbonylaminogruppe. Nach beendeter Umsetzung kann die Aminoschutzgruppe wieder in an sich bekannter Weise entfernt werden.

Die substituierten Acetophenone der Formeln I und II besitzen antivirale Aktivität und hemmen insbesondere die Replikation humaner Rhinoviren in Lungenzellen menschlicher Embryos oder in HeLa-Zellkulturen bei Konzentrationen von 0,006-1 µg/ml.

Ergebnisse der Untersuchungen auf antivirale Aktivität :

1. In vitro-Versuche

A) Hemmung des viralen cytopathogenen Effekts :

Eine Suspension von HeLa-Zellen ($6 \times 10^4$) wurde mit Rhinovirus HGP (Typ 2) ($3 \times 10^3$ koloniebildende Einheiten) gemischt und auf eine Mikrotestplatte gebracht, die die zu testenden Verbindungen in einer Verdünnungsreihe enthielt. Die Zellen wurden mit Eagel's Minimalmedium, enthaltend 2 % Kälberserum, 1 % Tryptosephosphatbrühe, 100 µg/ml Streptomycinsulfat und 20 Einheiten/ml Penicillin G, kultiviert. Nach 2-tägiger Kultivierung bei 33 °C wurde der virale cytopathogene Effekt mittels eines Mikroskops festgestellt.

Die Testergebnisse sind in der folgenden Tabelle 1 zusammengestellt. Es sind diejenigen Konzentrationen der getesteten Verbindungen angegeben, die eine Reduktion des viralen cytopathogenen Effektes um 50 %, verglichen mit einer Kontrollkultur, bewirken ($IC_{50}$).

Tabelle 1

| Verbindung | $IC_{50}$ (µg/ml) gegen Rhinovirus HGP |
|---|---|
| 2'-Hydroxy-4,4',6'-trimethoxychalkon | 0.01 |
| 2',4-Dihydroxy-4',6'-dimethoxychalkon | 0.3 - 1 |
| 4-(Benzyloxy)-2'-hydroxy-3,4',6'-trimethoxychalkon | 1 |
| 4-Ethoxy-2'-hydroxy-4',6'-dimethoxychalkon | 0.02 - 0.05 |
| 2',4-Dihydroxy-3,4',6'-trimethoxychalkon | 0.3 - 1 |
| 2'-Hydroxy-4',6'-dimethoxy-4-propoxychalkon | 0.1 |
| 2'-Hydroxy-4',6'-dimethoxychalkon | 0.1 - 0.3 |
| 2'-Hydroxy-4',6'-dimethoxy-4-methylchalkon | 0.01 |
| 2'-Hydroxy-4',6'-dimethoxy-3-(3-pyridyl)-acrylophenon | 0.6 |
| 2'-Hydroxy-4',6'-dimethoxy-3,4-(methylendioxy)-chalkon | 0.02 |
| 2'-Hydroxy-4',6'-dimethoxy-4-(methylthio)-chalkon | 0.03 |
| 4-(Allyloxy)-2'-hydroxy-4',6'-dimethoxychalkon | 0.03 - 0.1 |
| 4-(Dimethylamino)-2'-hydroxy-4',6'-dimethoxychalkon | 0.02 - 0.06 |

Tabelle 1 (Fortsetzung)

| Verbindung | $IC_{50}$ (µg/ml) gegen Rhinovirus HGP |
|---|---|
| 4-(Diethylamino)-2'-hydroxy-4',6'-dimethoxychalkon | 0.6 - 2 |
| 4-Chloro-2'-hydroxy-4',6'-dimethoxychalkon | 0.02 - 0.06 |
| 2',4,4'-Triethoxy-6'-hydroxychalkon | 0.25 |
| 2'-Hydroxy-4,4'-dimethoxychalkon | 0.3 - 1 |
| 2'-Hydroxy-4,4'-dimethoxy-6'-propoxychalkon | 0.06 |
| 2'-Hydroxy-6'-isopropoxy-4,4'-dimethoxychalkon | 0.1 - 0.2 |
| 2'-Ethoxy-6'-hydroxy-4,4'-dimethoxychalkon | 0.01 |
| 3-(2-Furyl)-2'-hydroxy-4',6'-dimethoxyacrylophenon | 0.06 - 0.2 |
| 2'-Hydroxy-4',6'-dimethoxy-3-(2-thienyl)-acrylophenon | 0.03 |
| 2'-Hydroxy-4',6'-dimethoxy-3-(3-methyl-2-thienyl)-acrylophenon | 0.1 |
| 2'-Hydroxy-4',6'-dimethoxy-3-(5-methyl-2-furyl)-acrylophenon | 0.03 |
| 2'-Hydroxy-4',6'-dimethoxy-3-(N-methyl-2-pyrrolyl)-acrylophenon | 0.1 |
| 4-Amino-2'-hydroxy-4',6'-dimethoxychalkon | 0.03 - 0.1 |
| 4-Cyano-2'-hydroxy-4',6'-dimethoxychalkon | 0.3 |
| 2'-Acetoxy-4,4',6'-trimethoxychalkon | 0.006 |
| 2',4,4'-Trimethoxy-6'-(propionyloxy)-chalkon | 0.01 |
| 2',4,4'-Trimethoxy-6'-(octadecanoyloxy)-chalkon | 0.05 - 0.1 |
| 2'-(Ethoxycarbonyloxy)-4,4',6'-trimethoxychalkon | 0.04 |
| 2',4,4'-Trimethoxy-6'-(nicotinoyloxy)-chalkon | 0.01 - 0.025 |
| 2',4,4'-Trimethoxy-6'-(pyrazinylcarbonyloxy)-chalkon | 0.03 - 0.1 |

Tabelle 1 (Fortsetzung)

| Verbindung | $IC_{50}$ (µg/ml) gegen Rhinovirus HGP |
|---|---|
| 2'-(L-Alanyloxy)-4,4',6'-trimethoxychalkon | 0.03 - 0.1 |
| 2'-(4-Carboxybutanoyloxy)-4,4',6'-trimethoxychalkon | 0.03 |
| 4-Ethoxy-2',4'-dimethoxy-6'-(propionyloxy)-chalkon | 0.03 ∿ 0.1 |
| 2',4'-Dimethoxy-6'-(propionyloxy)-4-propoxychalkon | 0.1 ∿ 0.3 |
| 2',4'-Dimethoxy-4-methyl-6'-(propionyloxy)-chalkon | 0.03 |
| 2',4'-Dimethoxy-4-(methylthio)-6'-(propionyloxy)-chalkon | 0.03 ∿ 0.1 |
| 4-(Allyloxy)-2',4'-dimethoxy-6'-(propionyloxy)-chalkon | 0.03 ∿ 0.1 |
| 2',4,4'-Triethoxy-6'-(propionyloxy)-chalkon | 0.1 ∿ 0.3 |
| 2',4'-Dimethoxy-6'-(propionyloxy)-chalkon | 0.3 ∿ 1 |
| 4-Chloro-2',4'-dimethoxy-6'-(propionyloxy)-chalkon | 0.1 ∿ 0.3 |
| 2'-Ethoxy-4,4'-dimethoxy-6'-(propionyloxy)-chalkon | 0.02 ∿ 0.06 |
| 2',4'-Dimethoxy-6'-(propionyloxy)-3-(2-thienyl)-acrylophenon | 0.1 ∿ 0.3 |
| 2',4'-Dimethoxy-3-(5-methyl-2-furyl)-6'-(propionyloxy)-acrylophenon | 0.03 ∿ 0.1 |
| 2',4'-Dimethoxy-3-(1-methylpyrrol-2-yl)-6'-(propionyloxy)-acrylophenon | 0.3 |
| 3-(2-Furyl)-2',4'-dimethoxy-6'-(propionyloxy)-acrylophenon | 0.1 ∿ 0.3 |
| 2',4'-Dimethoxy-3,4-(methylendioxy)-6'-(propionyloxy)-chalkon | 0.1 |
| 4,4'-Dimethoxy-2'-(propionyloxy)-6'-propoxychalkon | 0.03 ∿ 0.1 |
| 2'-Isopropoxy-4,4'-dimethoxy-6'-(propionyloxy)-chalkon | 0.01 ∿ 0.03 |
| 4'-Ethoxy-2'-hydroxy-4,6'-dimethoxy-chalkon | 0.002 ∿ 0.006 |

Tabelle 1 (Fortsetzung)

| Verbindung | $IC_{50}$ (µg/ml) gegen Rhinovirus HGP |
|---|---|
| 4,4'-Diethoxy-2'-hydroxy-6'-methoxy-chalkon | 0.006 ∿ 0.02 |
| 2'-Hydroxy-3,4',6'-trimethoxychalkon | 0.1 |
| 2',4-Diethoxy-6'-hydroxy-4'-methoxy-chalkon | 0.1 |
| 2'-Hydroxy-3,4',5,6'-tetramethoxychalkon | 0.3 |
| 2,2'-Dihydroxy-3,4',6'-trimethoxychalkon | 0.15 ∿ 0.5 |
| 4'-Ethoxy-2'-hydroxy-6'-methoxy-3-(5-methyl-2-furyl)-acrylophenon | 0.03 |
| 2'-Ethoxy-6'-hydroxy-4'-methoxy-3-(5-methyl-2-furyl)-acrylophenon | 0.04 |
| 2',4'-Diethoxy-6'-hydroxy-3-(5-methyl-2-furyl)-acrylophenon | 0.04 ∿ 0.12 |
| 2',4,4'-Trimethoxy-6'-(pivaloyloxy)-chalkon | 0.01 ∿ 0.03 |
| 2',4'-Diethoxy-6'-hydroxy-4-methoxy-chalkon | 0.03 ∿ 0.1 |
| 2',3-Dihydroxy-4,4',6'-trimethoxychalkon | 0.1 ∿ 0.3 |
| 2'-Hydroxy-3,4,4',6'-tetramethoxychalkon | 0.1 ∿ 0.3 |
| 2'-Hydroxy-2,4,4',6'-tetramethoxychalkon | 0.1 |
| 2'-Hydroxy-2,3,4',6'-tetramethoxychalkon | 0.1 |

B) Hemmung der viralen Replikation :

Der Effekt von 2'-Hydroxy-4,4',6'-trimethoxychalkon auf die Replikation von Rhinovirus HGP in HeLa-Zellen wurde bestimmt. Monomolekulare Schichten von HeLa-Zellen (4 × $10^5$), denen 2'-Hydroxy-4,4',6'-trimethoxychalkon verschiedener Konzentrationen zugesetzt wurde, wurden während 60 Minuten mit Rhinovirus HGP (4 × $10^4$ koloniebildende Einheiten) infiziert. Anschliessend wurden die Zellen mit Eagel's Minimalmedium, enthaltend 2 % Kälberserum, 1 % Tryptosephosphatbrühe, 100 µg/ml Streptomycinsulfat und 20 Einheiten/ml Penicillin G, gewaschen und weiter in diesem Medium gezüchtet. 2 Tage nach der Infektion wurde die Ausbeute an Viren im Kulturmedium bestimmt.

Die Ergebnisse, die in Figur 1 dargestellt sind, zeigen ganz klar, dass 2'-Hydroxy-4,4',6'-trimethoxychalkon die virale Replikation der Viren erheblich reduziert in Konzentrationen von 0,1-1,0 µg/ml, ohne irgend einen cytotoxischen Nebeneffekt.

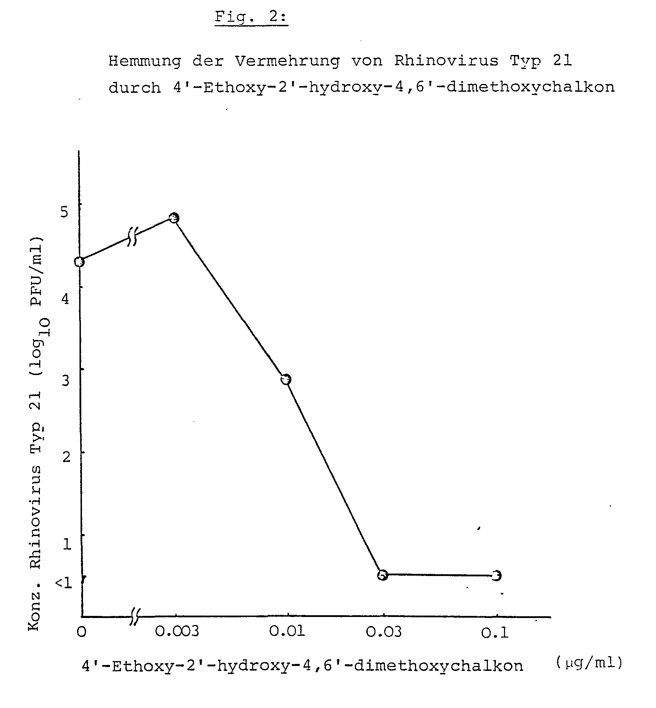

In derselben Weise wurde der Effekt von 4'-Ethoxy-2'-hydroxy-4,6'-dimethoxychalkon auf die Replikation von Rhinoviren Typ 21 in HeLa-Zellen getestet. Die Ergebnisse sind in Figur 2 zusammengestellt und zeigen, dass 4'-Ethoxy-2'-hydroxy-4,6'-dimethoxychalkon die virale Replikation in Konzentrationen von 0,03 µg/ml stark reduziert, ohne cytotoxische Symptome zu zeigen.

2. Antivirale in vivo-Aktivität

Die Aktivität der Verbindungen gegen letale Infektionen mit Coxsackie Virus B1 wurde an Mäusen getestet. DDY-Mäuse mit einem Gewicht von 15 g wurden intraperitoneal mit etwa der zehnfachen $LD_{50}$-

Dosis des Virus infiziert. Die infizierten Mäuse wurden entweder oral 0, 2, 5, 18, 24, 42, 48, 66 und 72 Stunden oder intraperitoneal 1, 2, 4, 18 und 28 Stunden nach der Infektion behandelt. Die überlebenden Tiere wurden bis zu 21 Tagen registriert.

Die Ergebnisse sind in der Tabelle 2 zusammengefasst. Nicht behandelte Tiere (Kontrollen) starben nach 3-5 Tagen nach der Infektion.

Die erfindungsgemässen Verbindungen werden sehr gut vertragen und zeigen keinerlei toxische Aktivität bei Konzentrationen, die 10 x bis 1 000 x höher sind als ihre Wirkungsdosis gegen Rhinovirus-Infektionen. Bei oraler Applikation wurden keine toxischen Symptome bei Dosierungen von 5 g/kg und darüber festgestellt. Die akuten Toxizitäten sind in der folgenden Tabelle 3 zusammengestellt.

Tabelle 2

Antivirale Aktivität gegen Coxsackievirus B1 an Mäusen

| Verbindung | Dosierung | Ueberlebende [%] |
|---|---|---|
| 2'-Hydroxy-4,4',6'-trimethoxychalkon | 40 mg/kg x 9 p.o.<br>20 mg/kg x 9 p.o. | 60<br>40 |
| 2'-Acetoxy-4,4',6'-trimethoxychalkon | 40 mg/kg x 9 p.o.<br>20 mg/kg x 9 p.o. | 70<br>40 |
| 2'-Hydroxy-4',6'-dimethoxy-3-(2-thienyl)-acrylophenon | 40 mg/kg x 9 p.o.<br>20 mg/kg x 9 p.o. | 70<br>30 |
| Kontrolle | - - | 20 |
| 2'-Acetoxy-4,4',6'-trimethoxychalkon | 40 mg/kg x 5 i.p. | 50 |
| 2',4,4'-Trimethoxy-6'-(propionyloxy)-chalkon | 40 mg/kg x 5 i.p.<br>20 mg/kg x 5 i.p. | 75<br>25 |
| Kontrolle | - - | 0 |

Tabelle 3

| Verbindung | $LD_{50}$ (mg/kg)[1)] | |
|---|---|---|
| | i.p.[2)] | p.o.[3)] |
| 2'-Hydroxy-4,4',6'-trimethoxychalkon | > 1,000 | > 5,000 |

Tabelle 3 (Fortsetzung)

| Verbindung | $LD_{50}$ (mg/kg)[1)] | |
|---|---|---|
| | i.p.[2)] | p.o.[3)] |
| 2'-Acetoxy-4,4',6'-trimethoxy-chalkon | > 1,000 | > 5,000 |
| 4-(Dimethylamino)-2'-hydroxy-4',6'-dimethoxychalkon | > 1,000 | > 5,000 |
| 2'-Hydroxy-4',6'-dimethoxy-3-(2-thienvl)-acrylophenon | > 1,000 | > 5,000 |
| 4-Chloro-2'-hydroxy-4',6'-dimethoxychalkon | > 1,000 | > 5,000 |
| 4-Ethoxy-2'-hydroxy-4',6'-dimethoxychalkon | > 1,000 | > 5.000 |
| 2'-Hydroxy-4',6'-dimethoxy-4-methylchalkon | > 1,000 | > 5,000 |
| 2'-Hydroxy-4'-6'-dimethoxy-3,4-(methylenedioxy)-chalkon | > 1,000 | > 5,000 |
| 2'-Hydroxy-4',6'-dimethoxy-4-(methylthio)-chalkon | > 1,000 | > 5,000 |
| 2'-Hydroxy-4',6'-dimethoxy-3-(3-methyl-2-thienyl)-acrylophenon | > 500 | > 1,500 |
| 2'-Hydroxy-4',6'-dimethoxy-3-(5-methyl-2-furyl)-acrylophenon | > 500 | > 2,000 |
| 2'-Hydroxy-4',6'-dimethoxy-3-(N-methyl-2-pyrrolyl)-acrylophenon | > 500 | > 1,000 |
| 4-Cyano-2'-hydroxy-4',6'-dimethoxychalkon | > 500 | > 1,000 |
| 2',4,4'-Trimethoxy-6'-(propionyloxy)-chalkon | > 500 | > 5,000 |
| 2',4,4'-Trimethoxy-6'-(octadecanoyloxy)-chalkon | > 500 | > 4,000 |
| 2'-(Ethoxycarbonyloxy)-4,4',6'-trimethoxychalkon | > 500 | > 4,000 |
| 2',4,4'-Trimethoxy-6'-(nicotinoyloxy)-chalkon | > 500 | > 4,000 |
| 2',4,4'-Trimethoxy-6'-(pyrazinylcarbonyloxy)-chalkon | > 500 | > 4,000 |
| 2'-(L-Alanyloxy)-4,4',6'-trimethoxychalkon | > 180 | > 1,000 |

Tabelle 3 (Fortsetzung)

| Verbindung | $LD_{50}$ (mg/kg)[1] | |
|---|---|---|
| | i.p.[2] | p.o.[3] |
| 2'-(4-Carboxybutanoyloxy)-4,4',6'-trimethoxychalkon | > 500 | > 2,000 |
| 2',4-Dihydroxy-4',6'-dimethoxy-chalkon | > 500 | > 3,200 |
| 4-(Benzyloxy)-2'-hydroxy-3,4',6'-trimethoxychalkon | > 500 | > 1,500 |
| 2'-Hydroxy-4',6'-dimethoxy-4-propoxychalkon | > 500 | > 5,000 |
| 2'-Hydroxy-4',6'-dimethoxychalkon | > 1,000 | > 5,000 |
| 4-(Allyloxy)-2'-hydroxy-4',6'-dimethyloxychalkon | > 500 | > 1,000 |
| 2',4,4'-Triethoxy-6'-hydroxy-chalkon | > 1,000 | > 3,000 |
| 2'-Hydroxy-4,4'-dimethoxychalkon | > 1,000 | > 2,500 |
| 2'-Hydroxy-4,4'-dimethoxy-6'-propoxychalkon | > 500 | > 1,500 |
| 2'-Hydroxy-6'-isopropoxy-4,4'-dimethoxychalkon | > 500 | > 2,700 |
| 2'-Ethoxy-6'-hydroxy-4.4'-dimethoxychalkon | > 500 | > 1,000 |
| 4-Ethoxy-2',4'-dimethoxy-6'-(propionyloxy)-chalkon | > 1,000 | > 5,000 |
| 2',4'-Dimethoxy-6'-propionyl-oxy)-4-propoxychalkon | > 1,000 | > 5,000 |
| 2',4'-dimethoxy-4-methyl-6'-(propionyloxy)-chalkon | > 1,000 | > 5,000 |
| 2',4'-Dimethoxy-4-(methylthio)-6'-(propionyloxy)-chalkon | > 1,000 | > 5,000 |
| 4-(Allyloxy)-2',4'-dimethoxy-6'-(propionyloxy)-chalkon | > 500 | > 1,000 |
| 2',4,4'-Triethoxy-6'-(pro-pionyloxy)-chalkon | > 1,000 | > 5,000 |
| 2',4'-Dimethoxy-6'-(propionyl-oxy)-chalkon | > 1,000 | > 5,000 |
| 4-Chloro-2',4'-dimethoxy-6'-(propionyloxy)-chalkon | > 1,000 | > 5,000 |

Tabelle 3 (Fortsetzung)

| Verbindung | $LD_{50}$ (mg/kg)[1) | |
|---|---|---|
| | i.p.[2)] | p.o.[3)] |
| 2'-Ethoxy-4,4'-dimethoxy-6'-(propionyloxy)-chalkon | > 1,000 | > 5,000 |
| 2',4'-Dimethoxy-6'-(propionyloxy)-3-(2-thienyl)-acrylophenon | > 1,000 | > 5,000 |
| 2',4'-Dimethoxy-3-(5-methyl-2-furyl)-6'-(propionyloxy)-acrylophenon | > 500 | > 2,000 |
| 2',4'-Dimethoxy-3-(1-methylpyrrol-2-yl)-6'-(propionyloxy)-acrylophenon | > 500 | > 5,000 |
| 3-(2-Furyl)-2',4'-dimethoxy-6'-(propionyloxy)-acrylophenon | > 500 | > 1,000 |
| 2',4'-Dimethoxy-3,4-(methylendioxy)-6'-(propionyloxy)-chalkon | > 500 | > 1,000 |
| 4,4'-Dimethoxy-2'-(propionyloxy)-6'-propoxychalkon | > 500 | > 1,000 |
| 2'-Isopropoxy-4,4'-dimethoxy-6'-(propionyloxy)-chalkon | > 500 | > 1,000 |
| 4'-Ethoxy-2'-hydroxy-4,6'-dimethoxychalkon | > 1,000 | > 5,000 |
| 4,4'-Diethoxy-2'-hydroxy-6'-methoxychalkon | > 500 | > 1,000 |
| 2'-Hydroxy-3,4',6'-trimethoxychalkon | > 500 | > 1,000 |
| 2',4-Diethoxy-6'-hydroxy-4'-methoxychalkon | > 500 | > 5,000 |
| 2'-Hydroxy-3,4',5,6'-tetramethoxychalkon | > 500 | > 5,000 |
| 2,2'-Dihydroxy-3,4',6'-trimethoxychalkon | > 500 | > 1,000 |
| 4'-Ethoxy-2'-hydroxy-6'-methoxy-3-(5-methyl-2-furyl)-acrylophenon | > 500 | > 1,000 |
| 2'-Ethoxy-6'-hydroxy-4'-methoxy-3-(5-methyl-2-furyl)-acrylophenon | > 500 | > 1,000 |

Tabelle 3 (Fortsetzung)

| Verbindung | $LD_{50}$ (mg/kg)[1)] | |
|---|---|---|
| | i.p.[2)] | p.o.[3)] |
| 2',4'-Diethoxy-6'-hydroxy-3-(5-methyl-2-furyl)-acrylophenon | > 500 | > 1,000 |
| 2',4,4'-Trimethoxy-6'-pivaloyloxychalkon | > 1,000 | > 5,000 |
| 2',4'-Diethoxy-6'-hydroxy-4-methoxychalkon | > 1,000 | > 2,000 |
| 2',3-Dihydro-4,4',6'-trimethoxychalkon | > 500 | > 1,000 |
| 2'-Hydroxy-3,4,4',6'-tetramethoxychalkon | > 500 | > 500 |
| 2'-Hydroxy-2,4,4',6'-tetramethoxychalkon | > 500 | > 1,000 |
| 2'-Hydroxy-2,3,4',6'-tetramethoxychalkon | > 500 | > 1,000 |

1) DDY-Mäuse mit einem Gewicht von 15-20 g wurden mit einer einfachen Dosis der jeweiligen Verbindung behandelt. Die überlebenden Tiere wurden am 21. Tag gezählt.

2) Verbindungen gelöst in Dimethylsulfoxid.

3) Verbindungen suspendiert in einer Lösung von 0,5% Carboxymethylcellulose.

Wie bereits erwähnt, können die Verbindungen der Formeln I und II als Medikamente gegen virale Infektionen, insbesondere gegen Erkältung, in Form pharmazeutischer Präparate verwendet werden.

Die pharmazeutischen Präparate enthalten mindestens eine der genannten viralen Verbindungen sowie ein physiologisch verträgliches pharmazeutisches Trägermaterial und können noch weitere pharmazeutisch aktive Verbindungen enthalten. Die pharmazeutischen Präparate für orale Applikationen können in fester Form, beispielsweise als Tabletten, Kapseln, Pillen, Puder und Granulate oder in flüssiger Form, wie Lösungen, Suspensionen, Sirupe und Elixiere, vorliegen. Präparate für parenterale Verabreichungen sind beispielsweise sterile Lösungen, Suspensionen oder Emulsionen. Präparate für topische Anwendung sind Lösungen, Emulsionen, mikronisierte Puder, Salben, Gurgelwässer, Pastillen und Aerosole.

Die pharmazeutischen Präparate können in einer solchen Menge verabreicht werden, dass die Konzentration der aktiven Wirkstoffe grösser ist als die minimale Hemmkonzentration für bestimmte zu behandelnde virale Infektionen. Die Dosierung hängt von der Art der Verabreichung, dem Alter, Gewicht und dem Zustand des Patienten ab und der speziellen zu behandelnden Krankheit. Im allgemeinen kann als Richtwert für die Behandlung von Erkältungen bei Erwachsenen eine Menge von 100-2 000 mg, 3-6 mal täglich bei oraler Behandlung und etwa 0,1-100 $\mu g/cm^2$, 3-6 mal täglich, bei topischer Anwendung empfohlen werden.

Die folgenden Beispiele erläutern die vorliegende Erfindung. Die Beispiele 2-4, 6-19, 21-25 und 27-31

betreffen Verbindungen der Formel II gemäss Anspruch 5.

Beispiel 1

Zu einer Lösung von 196 mg 2'-Hydroxy-4',6'-dimethoxyacetophenon und 150 mg Anisaldehyd in 3 ml Ethanol wurden unter Rühren 3 ml einer 50 %igen wässrigen Kaliumhydroxidlösung gegeben. Es wurde 3 Tage bei Raumtemperaur gerührt und dann in 30 ml kaltes Wasser gegossen. Das Gemisch wurde 3 mal mit je 30 ml Ethylacetat extrahiert. Die vereinigten Ethylacetatextrakte wurden mit Wasser gewaschen, über wasserfreiem Natriumsulfat getrocknet und unter vermindertem Druck eingeengt. Nach Umkristallisation aus Ethanol wurden 182 mg (Ausbeute 50 %) 2'-Hydroxy-4,4',6'-trimethoxychalkon in Form gelber Nadeln erhalten, Smp. 170,7 °C.

Analog wurden erhalten :

2',4-Dihydroxy-4',6-dimethoxychalkon ;
Smp. 194,7 °C (umkristallisiert aus Methanol).
4-(Benzyloxy)-2'-hydroxy-3,4',6'-trimethoxychalkon ;
Smp. 130,7 °C (Methanol).
2'-Hydroxy-4',6'-dimethoxy-3,4-(methylendioxy)-chalkon ;
Smp. 161,7 °C (Methanol).
4-Chlor-2'-hydroxy-4',6'-dimethoxychalkon ;
Smp. 167,3 °C (Methanol).
2'-Hydroxy-4,4'-dimethoxychalkon ;
Smp. 108,0 °C (Ethanol).

Beispiel 2

In zu Beispiel 1 analoger Weise wurden die folgenden substituierten Acetophenone aus den entsprechenden Acetophenonen und Aldehyden erhalten :

4-Ethoxy-2'-hydroxy-4',6'-dimethoxychalkon ;
Smp. 136,5 °C (Methanol).
2',4-Dihydroxy-3,4',6'-trimethoxychalkon ;
Smp. 176,8 °C (Methanol).
2'-Hydroxy-4',6'-dimethoxy-4-propoxychalkon ;
Smp. 96,7 °C (Methanol).
2'-Hydroxy-4',6'-dimethoxychalkon ;
Smp. 85,5 °C (Methanol).
2'-Hydroxy-4',6'-dimethoxy-4-methylchalkon ;
Smp. 129,5 °C (Methanol).
2'-Hydroxy-4',6'-dimethoxy-3-(3-pyridyl)-acrylophenon ;
Smp. 158,3 °C (Methanol).
2'-Hydroxy-4',6'-dimethoxy-4-(methylthio)-chalkon ;
Smp 134,6 °C (Methanol).
4-(Allyloxy)-2'-hydroxy-4',6'-dimethoxychalkon ;
Smp. 112,3 °C (Methanol).
4-(Dimethylamino)-2'-hydroxy-4',6'-dimethoxychalkon ;
Smp. 196,7 °C (Methanol).
4-(Diethylamino)-2'-hydroxy-4',6'-dimethoxychalkon ;
Smp. 121,8 °C (Ether/Petrolether).
2',4,4'-Triethoxy-6'-hydroxychalkon ;
Smp. 131,0 °C (Methanol).
2'-Hydroxy-4,4'-dimethoxy-6'-propoxychalkon ;
Smp. 116,7 °C (Ethanol).
2'-Hydroxy-6'-isopropoxy-4,4'-dimethoxychalkon ;
Smp. 112,8 °C (Ethanol).
2'-Ethoxy-6'-hydroxy-4,4'-dimethoxychalkon ;
Smp. 123,6 °C (Methanol).
3-(2-Furyl)-2'-hydroxy-4',6'-dimethoxyacrylophenon ;
Smp. 98,4 °C (Methanol).
2'-Hydroxy-4',6'-dimethoxy-3-(2-thienyl)-acrylophenon ;
Smp. 124,4 °C (Methanol).
2'-Hydroxy-4',6'-dimethoxy-3-(3-methyl-2-thienyl)-acrylophenon ;
Smp. 124,4 °C (Methanol).
2'-Hydroxy-4',6'-dimethoxy-3-(5-methyl-2-furyl)-acrylophenon ;
Smp. 103,1 °C (Methanol).

2'-Hydroxy-4',6'-dimethoxy-3-(1-methyl-pyrrol-2-yl)-acrylophenon ; Smp. 139,8 °C (Methanol).

Beispiel 3

Ein Gemisch aus 98 mg 2'-Hydroxy-4',6'-dimethoxyacetophenon, 85 mg 4-Acetamidobenzaldehyd und 1 g Kaliumhydroxid in 4 ml 50 %igem wässrigem Ethanol wurde 3 Tage bei Raumtemperatur gerührt. Das Gemisch wurde in 30 ml kaltes Wasser gegossen und 3 x mit je 30 ml Ethylacetat extrahiert. Die vereinigten Extrakte wurden mit Wasser gewaschen, über wasserfreiem Natriumsulfat getrocknet und unter vermindertem Druck eingeengt. Der Rückstand wurde durch präparative Dünnschichtchromatographie an Kieselgel mit Cyclohexan/Ethylacetat (1 : 1, v/v) gereinigt. Es wurden 12 mg 4-Amino-2'-hydroxy-4',6'-dimethoxychalkon in Form eines gelben Pulvers erhalten : Rf 0,22 ; $^1$H-NMR-Spektrum (in $CDCl_3$) : δ 3,6(2H), 3,72(3H), 3,82(3H), 5,90(1H), 6,06(1H), 6,56(1H), 6,70(1H), 7,50(1H), 7,62(1H) und 14,4 ppm (1H).

Beispiel 4

Zu einer Lösung von 196 mg 2'-Hydroxy-4',6'-dimethoxyacetophenon und etwa 100 mg frisch hergestelltem Natriumethoxid in 5 ml absolutem Ethanol wurden unter Rühren 131 mg 4-Cyanobenzaldehyd gegeben. Das Gemisch wurde 5 Stunden bei Raumtemperatur gerührt. Nach Zusatz von 30 ml Wasser wurde das Gemisch mit verdünnter Salzsäure auf pH 4 eingestellt und 3 x mit jeweils 30 ml Ethylacetat extrahiert. Die vereinigten Extrakte wurden mit Wasser gewaschen, über wasserfreiem Natriumsulfat getrocknet und unter vermindertem Druck eingeengt. Es wurden 108 mg eines Feststoffes erhalten, der nach Umkristallisation aus Methanol 78 mg (25 %) 4-Cyan-2'-hydroxy-4',6'-dimethoxychalkon in Form gelber Kristalle lieferte ; Smp. 230,9 °C.

Beispiel 5

Zu einer eiskalten Lösung von 300 ml 2'-Hydroxy-4,4',6'-trimethoxychalkon in 5 ml Pyridin wurden 0,1 ml Acetylchlorid gegeben. Das Gemisch wurde 1 Stunde bei Raumtemperatur gerührt, nach Entfernung des Lösungsmittels unter vermindertem Druck mit 10 ml Eiswasser und 30 ml Chloroform behandelt und geschüttelt. Die Chloroformschicht wurde abgetrennt, 3 x mit je 10 ml Wasser gewaschen, über wasserfreiem Natriumsulfat getrocknet und unter vermindertem Druck eingeengt. Der erhaltene Rückstand wurde aus Ethylacetat/Hexan umkristallisiert und lieferte 310 mg (92 %) 2'-Acetoxy-4,4',6'-trimethoxychalkon in Form farbloser Nadeln ; Smp. 105-107 °C.

Beispiel 6

Eine Lösung von 500 mg 2'-Hydroxy-4,4',6'-trimethoxychalkon und 0,15 ml Propionylchlorid in 5 ml Pyridin wurde 6 Stunden bei Raumtemperatur gerührt. Nach Entfernung des Lösungsmittels wurde der Rückstand mit 10 ml Eiswasser und 30 ml Chloroform behandelt. Das Gemisch wurde geschüttelt, die Chloroformschicht abgetrennt, 3 x mit je 10 ml Wasser gewaschen, über wasserfreiem Natriumsulfat getrocknet und unter vermindertem Druck eingeengt. Der ölige Rückstand wurde mit einer geringen Menge Hexan gewaschen und der erhaltene feste Rückstand aus Ethanol/Hexan umkristallisiert. Es wurden 530 mg (90 %) 2',4,4'-Trimethoxy-6'-(propionyloxy)-chalkon in Form hellgelber Nadeln erhalten ; Smp. 117-118 °C.

Beispiel 7

Eine Lösung von 120 mg 2'-Hydroxy-4,4',6'-trimethoxychalkon und 210 mg Stearinsäureanhydrid in 10 ml Pyridin wurde 8 Stunden bei 105 °C gerührt und anschliessend eingeengt. Dem öligen Rückstand wurden 30 ml Ethylacetat und 30 ml gesättigte wässrige Natriumbicarbonatlösung zugesetzt. Das Gemisch wurde geschüttelt, woraufhin sich ein fester Niederschlag von Natriumstearat bildete. Nach Entfernung des Niederschlages durch Filtration wurde die Ethylacetat-Schicht abgetrennt, mit Wasser gewaschen, über wasserfreiem Natriumsulfat getrocknet und eingeengt. Umkristallisation des Rückstandes aus Methanol lieferte 200 mg (91 %) 2',4,4'-Trimethoxy-6'-(octadecanoyloxy)-chalkon in Form hellgelber Nadeln ; Smp. 65-66 °C.

Beispiel 8

Zu einer Lösung von 314 mg 2'-Hydroxy-4,4',6'-trimethoxychalkon in 5 ml Pyridin wurden tropfenweise 216 mg Chlorameisensäureethylester gegeben. Nach 30 minütigem Rühren bei Raumtemperatur wurde das Gemisch in 30 ml Wasser gegossen. Das Gemisch wurde 3 x mit je 30 ml Chloroform extrahiert, die vereinigten Extrakte wurden mit Wasser gewaschen, über Natriumsulfat getrocknet und eingeengt. Umkristallisation des öligen Rückstandes aus Methanol lieferte 202 g (53 %)

2'-(Ethoxycarbonyloxy)-4,4',6'-trimethoxy-chalkon in Form hellgelber Prismen ; Smp. 107 °C.

Beispiel 9

Eine Lösung von 1 g 2'-Hydroxy-4,4',6'-trimethoxychalkon und 0,6 g Nicotinoylchlorid-hydrochlorid in 20 ml Pyridin wurde 4 Stunden bei Raumtemperatur gerührt. Nach Entfernung des Lösungsmittels und Zusatz von 50 ml gesättigter wässriger Natriumbicarbonatlösung wurde das Gemisch mit 100 ml Ethylacetat extrahiert. Der Extrakt wurde mit Wasser gewaschen, über Natriumsulfat getrocknet und unter vermindertem Druck eingeengt. Umkristallisation des öligen Rückstandes aus Methanol/Hexan lieferte 1,1 g (83 %) 2',4,4'-Trimethoxy-6'-(nicotinoyloxy)-chalkon in Form hellgelber Nadeln ; Smp. 55-60 °C.

Beispiel 10

Zu einer Lösung von 1 g 2'-Hydroxy-4,4',6'-trimethoxychalkon und 592 ml Pyrazincarbonsäure in 40 ml Pyridin wurden tropfenweise 0,46 ml Thionylchlorid gegeben. Das Gemisch wurde 2 Stunden bei Raumtemperatur gerührt und nach Zusatz von Eiswasser 3 x mit je 50 ml Ethylacetat extrahiert. Die vereinigten Extrakte wurden mit Wasser gewaschen, über Natriumsulfat getrocknet und unter vermindertem Druck eingeengt. Umkristallisation des kristallinen Rückstandes aus Aceton lieferte 860 mg (64 %) 2',4,4'-Trimethoxy-6'-(pyrazinylcarbonyloxy)-chalkon in Form hellgelber Kristalle ; Smp. 163-164 °C.

Beispiel 11

Zu einer gut gekühlten Lösung von 0,2 g 2'-Hydroxy-4,4',6'-trimethoxychalkon und N-Benzyloxycarbonyl-L-alanin in 5 ml Pyridin wurden 0,2 ml Thionylchlorid gegeben. Nach 3-stündigem Rühren bei − 15 bis − 20 °C und Zugabe von 25 ml Eiswasser wurde das Gemisch mit 50 ml Chloroform extrahiert. Der Extrakt wurde über Natriumsulfat getrocknet und unter vermindertem Druck eingeengt. Der Rückstand wurde in einer geringen Menge Chloroform gelöst und auf eine Kieselgelsäule (1 × 30 cm) gegeben. Nach Elution mit chloroform und Entfernung des Lösungsmittels wurden 200 ml eines hellgelben Pulvers erhalten. Umkristallisation aus Methanol/Hexan lieferte 150 mg 2'-(N-Benzyloxycarbonyl-L-alanyloxy)-4,4',6'-trimethoxychalkon in Form hellgelber Nadeln ; Smp. 163-164 °C. Dieses Material wurde in 5 ml Essigsäure, enthaltend 25 % HBr, gelöst. Nach 20-minütigem Stehen bei Raumtemperatur wurde die Lösung lyophilisiert und lieferte 120 mg eines Puders, der 3 x mit je 5 ml Chloroform gewaschen wurde und 110 ml (20 %) 2'-(L-Alanyloxy)-4,4',6'-trimethoxychalkon-hydrobromid in Form eines gelben Pulvers lieferte : Smp. 180-185 °C (Zers.).

Beispiel 12

76 mg Natriumhydrid (Reinheit 60 %) wurden unter Rühren zu einer eisgekühlten Lösung von 500 mg 2'-Hydroxy-4,4',6'-trimethoxychalkon in 20 ml Tetrahydrofuran gegeben. Nach 10 Minuten wurde eine Lösung von 218 mg Glutarsäureanhydrid in 3 ml Tetrahydrofuran zugesetzt. Das Gemisch wurde 2 Stunden bei Zimmertemperatur gerührt und unter vermindertem Druck eingeengt. Der Rückstand wurde in 50 ml Ethylacetat gelöst und die Lösung mit 50 ml 10 %igem wässrigem Natriumbicarbonat extrahiert. Der Extrakt wurde mit Salzsäure angesäuert und mit 50 ml Chloroform geschüttelt. Der Chloroformextrakt wurde dann mit Wasser gewaschen, über Natriumsulfat getrocknet und unter vermindertem Druck eingeengt. Umkristallisation des Rückstandes aus Benzol lieferte 75 ml (11 %) 2'-(4-Carboxybutanoyloxy)-4,4',6'-trimethoxychalkon in Form hellgelber Kristalle ; Smp. 131-132 °C.

Beispiel 13

Ein Gemisch aus 328 mg 4-Ethoxy-2'-hydroxy-4',6'-dimethoxychalkon, 0,3 ml Propionsäureanhydrid und 20 mg Natriumpropionat wurde 2 Stunden auf 130 °C erhitzt. Das erhaltene hellgelbe Oel wurde in etwa 70 ml Eiswasser gegossen und das Gemisch wurde 2 x mit je 70 ml Dichlormethan extrahiert. Die vereinigten Extrakte wurden mit Wasser gewaschen, über Natriumsulfat getrocknet und eingeengt. Umkristallisation des Rückstandes aus Methanol lieferte 267 mg (76 %) 4-Ethoxy-2',4'-Dimethoxy-6'-(propionyloxy)-chalkon in Form hellgelber Kristalle ; Smp. 104,7 °C.

Beispiel 14

In zu Beispiel 13 analoger Weise wurde aus 2'-Hydroxy-4',6'-dimethoxy-4-propoxychalkon 2',4'-Dimethoxy-6'-(propionyloxy)-4-propoxychalkon erhalten ; Smp. 102,5 °C.

Beispiel 15

In zu Beispiel 13 analoger Weise wurde aus 2'-Hydroxy-4',6'-dimethoxy-4-methylchalkon 2',4'-Dimethoxy-4-methyl-6'-(propionyloxy)-chalkon erhalten ; Smp. 156,0 °C.

Beispiel 16

In zu Beispiel 13 analoger Weise wurde aus 2'-Hydroxy-4',6'-dimethoxy-4-(methylthio)-chalkon 2',4'-Dimethoxy-4-(methylthio)-6'-(propionyloxy)-chalkon erhalten ; Smp. 108,4 °C.

Beispiel 17

In zu Beispiel 13 analoger Weise wurde aus 4-(Allyloxy)-2'-hydroxy-4',6'-dimethoxychalkon 4-(Allyloxy)-2',4'-dimethoxy-6'-(propionyloxy)-chalkon erhalten ; Smp. 83,5 °C.

Beispiel 18

In zu Beispiel 13 analoger Weise wurde aus 2',4,4'-Triethoxy-6'-hydroxychalkon 2',4,4'-Triethoxy-6'-(propionyloxy)-chalkon erhalten ; Smp. 84,5 °C.

Beispiel 19

In zu Beispiel 13 analoger Weise wurde aus 2'-Hydroxy-4',6'-dimethoxychalkon 2',4'-Dimethoxy-6'-(propionyloxy)-chalkon erhalten ; Smp. 115,5°-117,0 °C.

Beispiel 20

In zu Beispiel 13 analoger Weise wurde aus 4-Chlor-2'-hydroxy-4',6'-dimethoxychalkon 4-Chlor-2',4'-dimethoxy-6'-(propionyloxy)-chalkon erhalten ; Smp. 136,0 °C.

Beispiel 21

In zu Beispiel 13 analoger Weise wurde aus 2'-Ethoxy-6'-hydroxy-4,4'-dimethoxychalkon 2'-Ethoxy-4,4'-dimethoxy-6'-(propionyloxy)-chalkon erhalten ; Smp. 77,5 °C (Benzol/Hexan).

Beispiel 22

In zu Beispiel 13 analoger Weise wurde aus 2'-Hydroxy-4',6'-dimethoxy-3-(2-thienyl)-acrylophenon 2',4'-Dimethoxy-6'-(propionyloxy)-3-(2-thienyl)-acrylophenon erhalten ; Smp. 127,1 °C.

Beispiel 23

In zu Beispiel 13 analoger Weise wurde aus 2'-Hydroxy-4',6'-dimethoxy-3-(5-methyl-2-furyl)-acrylophenon 2',4'-Dimethoxy-3-(5-methyl-2-furyl)-6'-(propionyloxy)-acrylophenon erhalten ; Smp. 72,5 °C.

Beispiel 24

In zu Beispiel 13 analoger Weise wurde aus 2'-Hydroxy-4',6'-dimethoxy-3-(1-methylpyrrol-2-yl)-acrylophenon 2',4'-Dimethoxy-3-(1-methylpyrrol-2-yl)-6'-(propionyloxy)-acrylophenon erhalten ; Smp. 107,6 °C.

Beispiel 25

In zu Beispiel 13 analoger Weise wurde aus 3-(2-Furyl)-2'-hydroxy-4',6'-dimethoxyacrylophenon 3-(2-Furyl)-2',4'-dimethoxy-6'(propionyloxy)-acrylophenon erhalten ; Smp. 93,6 °C.

Beispiel 26

In zu Beispiel 13 analoger Weise wurde aus 2'-Hydroxy-4',6'-dimethoxy-3,4-(methylendioxy)-chalkon 2',4'-Dimethoxy-3,4-(methylendioxy)-6'-(propionyloxy)-chalkon erhalten ; Smp. 134,5 °C.

Beispiel 27

In zu Beispiel 13 analoger Weise wurde aus 2'-Hydroxy-4,4'-dimethoxy-6'-propoxychalkon 4,4'-Dimethoxy-2'-(propionyloxy)-6'-propoxychalkon erhalten ; $^1$H-NMR-Spektrum (in $CDCl_3$) : δ 0,89(3H),

1,13(3H), 1,70(2H), 2,45(2H), 3,80(6H), 3,88(2H), 6,23(1H), 6,36(1H), 6,73(1H), 6,74(2H), 7,37(1H) und 7,46 ppm (2H).

Beispiel 28

In zu Beispiel 13 analoger Weise wurde aus 2'-Hydroxy-6'-isopropoxy-4,4'-dimethoxychalkon 2'-Isopropoxy-4,4'-dimethoxy-6'-(propionyloxy)-chalkon erhalten ; $^1$H-NMR-Spektrum (in $CDCl_3$) : δ 1,22(9H), 2,44(2H), 3,80(6H), 4,52(1H), 6,23(1H), 6,33(1H), 6,83(1H), 6,84(2H), 7,37(1H) und 7,45 ppm (2H).

Beispiel 29

Zu einer Lösung von 440 mg 4'-Ethoxy-2'-hydroxy-6'-methoxyacetophenon und 598 mg Anisaldehyd in 20 ml Ethanol wurden unter Rühren 15 ml einer 15 %igen wässrigen Natriumhydroxidlösung gegeben. Nachdem 3 Tage bei Zimmertemperatur gerührt wurde, wurde das Gemisch unter Kühlen mit 6N Salzsäure neutralisiert. Der erhaltene kristalline Niederschlag wurde abfiltriert, mit Wasser gewaschen und aus Ethanol umkristallisiert. Es wurden 945 mg (72 %) 4'-Ethoxy-2'-hydroxy-4,6'-dimethoxychalkon in Form gelber Nadeln erhalten ; Smp. 122,0 °C.

Beispiel 30

In zu Beispiel 29 analoger Weise wurden unter Verwendung der entsprechenden Acetophenone und Aldehyde anstelle von 4'-Ethoxy-2'-hydroxy-6'-methoxyacetophenon und Anisaldehyd die folgenden substituierten Acetophenone erhalten. Alle Verbindungen wurden aus Methanol umkristallisiert mit Ausnahme von 4,4'-Diethoxy-2'-hydroxy-6'-methoxychalkon, das aus Ethanol umkristallisiert wurde.

4,4'-Diethoxy-2'-hydroxy-6'-methoxychalkon ;
Smp. 127,2 °C.
2'-Hydroxy-3,4',6'-trimethoxychalkon ;
Smp. 97,6 °C.
2',4-Diethoxy-6'-hydroxy-4'-methoxychalkon ;
Smp. 134,5 °C.
4'-Ethoxy-2'-hydroxy-6'-methoxy-3-(5-methyl-2-furyl)-acrylophenon ;
Smp. 115,0 °C.
2'-Ethoxy-6'-hydroxy-4'-methoxy-3-(5-methyl-2-furyl)-acrylophenon ;
Smp. 96,1 °C.
2',4'-Diethoxy-6'-hydroxy-3-(5-methyl-2-furyl)-acrylophenon ;
Smp. 140,9 °C.
2',4'-Diethoxy-6'-hydroxy-4-methoxychalkon ;
Smp. 137,0 °C.

Beispiel 31

Zu einer Lösung von 300 mg 2'-Hydroxy-4,4',6'-trimethoxychalkon in 12 ml Pyridin wurden unter Rühren 65 mg p-(Dimethylamino)-pyrimidin und 165 mg Pivaloylchlorid gegeben. Nach 4-stündigem Rühren bei Raumtemperatur wurde das Reaktionsgemisch in 30 ml Eiswasser gegossen. Die Mischung wurde mit Salzsäure angesäuert und mit 50 ml Ethylacetat extrahiert. Der Ethylacetat-Extrakt wurde nacheinander mit 0,2N Salzsäure, 10 %igem wässrigem Natriumcarbonat und Wasser gewaschen, über wasserfreiem Natriumsulfat getrocknet und eigeengt. Es wurden 440 mg 2',4,4'-Trimethoxy-6'-(pivaloyloxy)-chalkon erhalten ; $^1$H-NMR (in $CDCl_3$) : δ 1,20(9H), 3,78(3H), 3,83(6H), 6,25(1H), 6,40(1H), 6,80(1H), 6,85(2H), 7,10(1H) und 7,45 ppm (2H).

Beispiel 32

In zu Beispiel 29 analoger Weise wurden unter Verwendung der entsprechenden Acetophenone und Aldehyde anstelle von 4'-Ethoxy-2'-hydroxy-6'-methoxyacetophenon und Anisaldehyd die folgenden substituierten Acetophenone erhalten. Alle Verbindungen wurden aus Methanol umkristallisiert.

2'-Hydroxy-3,4',5,6'-tetramethoxychalkon ;
Smp. 164,2 °C.
2,2'-Dihydroxy-3,4',6'-trimethoxychalkon ;
Smp. 185,2 °C.
2',3-Dihydroxy-4,4',6'-trimethoxychalkon ;
Smp. 199,0 °C.
2'-Hydroxy-3,4,4',6'-tetramethoxychalkon ;
Smp. 158,9 °C.

2'-Hydroxy-2,4,4',6'-tetramethoxychalkon ; Smp. 153,0 °C.

2'-Hydroxy-2,3,4',6'-tetramethoxychalkon ; Smp. 125,9 °C.

**Ansprüche** (für die Vertragsstaaten : BE, CH, DE, FR, GB, IT, LU, NL, SE)

1. Substituierte Acetophenone der Formel

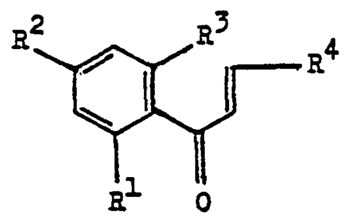

(I)

worin

$R^1$ Hydroxy, ein von einer aliphatischen Mono- oder Dicarbonsäure mit 2-18 C-Atomen oder einer heterocyclischen Carbonsäure, die Stickstoff enthält, abgeleitetes Acyloxy ; oder Aminoacyloxy ;

$R^2$ niederes Alkoxy,

$R^3$ Wasserstoff oder niederes Alkoxy ; und

$R^4$ einen Phenylrest, der durch einen oder mehrere Substituenten aus der Gruppe niederes Alkyl, niederes Alkoxy, Benzyloxy, Allyloxy, Alkylthio, Dialkylamino, Amino, Cyano, Hydroxy, Halogen und Alkylendioxy substituiert sein kann, einen gegebenenfalls durch niederes Alkyl substituierten Pyridyl-, Furyl-, Thienyl- oder Pyrrolyl-Rest darstellen, zur therapeutischen Behandlung des menschlichen oder tierischen Körpers gegen Viren.

2. Die Verbindung 4'-Ethoxy-2'-hydroxy-4,6'-dimethoxychalkon zur therapeutischen Behandlung des menschlichen oder tierischen Körpers gegen Viren.

3. Pharmazeutische Präparate, dadurch gekennzeichnet, dass sie eine Verbindung der Formel

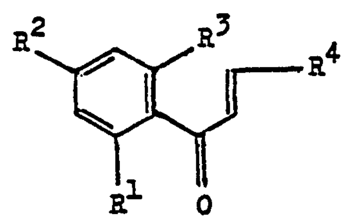

(I)

worin

$R^1$ Hydroxy, von einer aliphatischen Mono- oder Di-Carbonsäure mit 2-18 C-Atomen oder einer heterocyclischen Carbonsäure, die Stickstoff enthält, abgeleitetes Acyloxy ; oder Aminoacyloxy ;

$R^2$ niederes Alkoxy,

$R^3$ Wasserstoff oder niederes Alkoxy ; und

$R^4$ einen Phenylrest, der durch einen oder mehrere Substituenten aus der Gruppe niederes Alkyl, niederes Alkoxy, Benzyloxy, Allyloxy, Alkylthio, Dialkylamino, Amino, Cyano, Hydroxy, Halogen und Alkylendioxy substituiert sein kann, einen gegebenenfalls durch niederes Alkyl substituierten Pyridyl-, Furyl-, Thienyl- oder Pyrrolyl-Rest darstellen und ein Trägermaterial enthalten.

4. Pharmazeutische Präparate gemäss Anspruch 3, dadurch gekennzeichnet, dass sie 4'-Ethoxy-2'-hydroxy-4,6'-dimethoxychalkon enthalten.

5. Substituierte Acetophenone der allgemeinen Formel

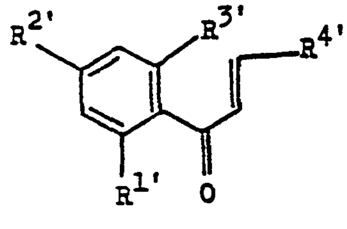

(II)

worin

$R^{1'}$ Hydroxy, von einer aliphatischen Mono- oder Dicarbonsäure mit 2-18 C-Atomen oder einer heterocyclischen Carbonsäure, die Stickstoff enthält, abgeleitetes Acyloxy ; oder Aminoacyloxy ;

$R^{2'}$ niederes Alkoxy ;

$R^{3'}$ niederes Alkoxy ; und

$R^{4'}$ einen Phenylrest, der durch einen oder mehrere Substituenten aus der Gruppe niederes Alkyl, niederes Alkoxy, Allyloxy, Alkylthio, Dialkylamino, Amino und Cyano substituiert sein kann, durch niederes Alkyl substituiertes Furyl oder gegebenenfalls durch niederes Alkyl substituiertes Pyridyl, Thienyl oder Pyrrolyl darstellen, mit den Einschränkungen, dass, wenn $R^{4'}$ p-Methoxyphenyl ist, $R^{1'}$ keinen Acetoxyrest darstellt, und dass $R^{2'}$ und $R^{3'}$ nicht gleichzeitig Methoxy oder Ethoxy sind, wenn $R^{1'}$ Hydroxy und $R^{4'}$ Methoxyphenyl oder Dimethoxyphenyl darstellen.

6. 4'-Ethoxy-2'-hydroxy-4,6'-dimethoxychalkon.

7. Verfahren zur Herstellung substituierter Acetophenone der allgemeinen Formel

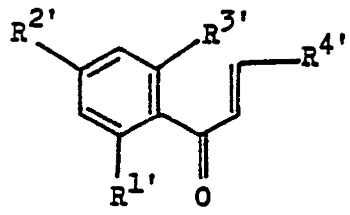

(II)

worin

$R^{1'}$ Hydroxy, von einer aliphatischen Mono- oder Dicarbonsäure mit 2-18 C-Atomen oder einer heterocyclischen Carbonsäure, die Stickstoff enthält, abgeleitetes Acyloxy ; oder Aminoacyloxy ;

$R^{2'}$ niederes Alkoxy ;

$R^{3'}$ niederes Alkoxy ; und

$R^{4'}$ einen Phenylrest, der durch einen oder mehrere Substituenten aus der Gruppe niederes Alkyl, niederes Alkoxy, Allyloxy, Alkylthio, Dialkylamino, Amino und Cyano substituiert sein kann, durch niederes Alkyl substituiertes Furyl oder gegebenenfalls durch niederes Alkyl substituiertes Pyridyl, Thienyl oder Pyrrolyl darstellen, mit den Einschränkungen, dass, wenn $R^{4'}$ p-Methoxyphenyl ist, $R^{1'}$ keinen Acetoxyrest darstellt, und dass $R^{2'}$ und $R^{3'}$ nicht gleichzeitig Methoxy oder Ethoxy sind, wenn $R^{1'}$ Hydroxy und $R^{4'}$ Methoxyphenyl oder Dimethoxyphenyl darstellen, dadurch gekennzeichnet, dass man

a) zwecks Herstellung von Verbindungen der Formel II mit $R^{1'}$ = Hydroxy eine Verbindung der Formel

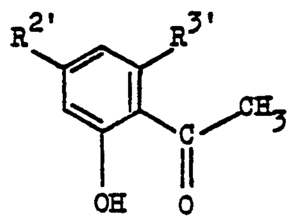

(III)

worin

$R^{2'}$ und $R^{3'}$ wie oben definiert sind, mit einem Aldehyd der Formel

$$R^{4'}\text{—CHO} \quad \text{(IV)}$$

worin

$R^{4'}$ wie oben definiert ist, in einem organischen Lösungsmittel, in Gegenwart eines basischen Katalysators, umsetzt oder

b) zwecks Herstellung von Verbindungen der Formel II, in denen $R^{1'}$ einen wie oben definierten Acyloxyrest oder Aminoacyloxyrest darstellt, eine Verbindung der Formel

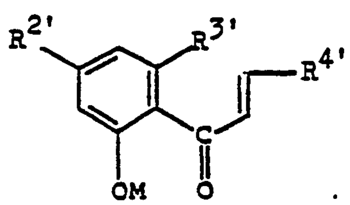

(V)

worin

$R^{2'}$, $R^{3'}$ und $R^{4'}$ wie oben definiert sind und

M Wasserstoff oder ein Alkalimetall bedeutet, mit einem Säureanhydrid oder Säurehalogenid einer aliphatischen Mono- oder Dicarbonsäure mit 2-18 C-Atomen, einer heterocyclischen Carbonsäure, die Stickstoff enthält, oder einer Aminosäure in einem organischen Lösungsmittel umsetzt.

**Anspruch** (für den Vertragsstaat AT)

1. Verfahren zur Herstellung substituierter Acetophenone der allgemeinen Formel

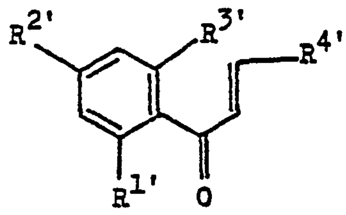

(II)

worin

$R^{1'}$ Hydroxy, von einer aliphatischen Mono- oder Dicarbonsäure mit 2-18 C-Atomen oder einer heterocyclischen Carbonsäure, die Stickstoff enthält, abgeleitetes Acyloxy ; oder Aminoacyloxy ;

$R^{2'}$ niederes Alkoxy ;

$R^{3'}$ niederes Alkoxy ; und

$R^{4'}$ einen Phenylrest, der durch einen oder mehrere Substituenten aus der Gruppe niederes Alkyl, niederes Alkoxy, Allyloxy, Alkylthio, Dialkylamino, Amino und Cyano substituiert sein kann, durch niederes Alkyl substituiertes Furyl oder gegebenenfalls durch niederes Alkyl substituiertes Pyridyl, Thienyl oder Pyrrolyl darstellen, mit den Einschränkungen, dass, wenn $R^{4'}$ p-Methoxyphenyl ist, $R^{1'}$ keinen Acetoxyrest darstellt, und dass $R^{2'}$ und $R^{3'}$ nicht gleichzeitig Methoxy oder Ethoxy sind, wenn $R^{1'}$ Hydroxy und $R^{4'}$ Methoxyphenyl oder Dimethoxyphenyl darstellen, dadurch gekennzeichnet, dass man

a) zwecks Herstellung von Verbindungen der Formel II mit $R^{1'}$ = Hydroxy eine Verbindung der Formel

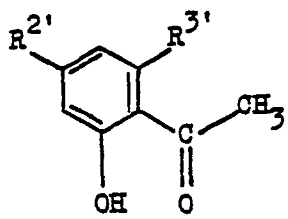

(III)

worin

$R^{2'}$ und $R^{3'}$ wie oben definiert sind, mit einem Aldehyd der Formel

$$R^{4'}—CHO \quad (IV)$$

worin

$R^{4'}$ wie oben definiert ist, in einem organischen Lösungsmittel, in Gegenwart eines basischen Katalysators, umsetzt oder

b) zwecks Herstellung von Verbindungen der Formel II, in denen $R^{1'}$ einen wie oben definierten Acyloxyrest oder Aminoacyloxyrest darstellt, eine Verbindung der Formel

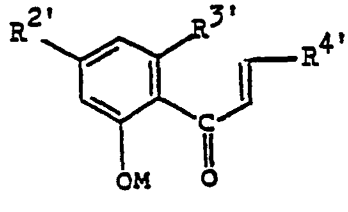

(V)

worin

$R^{2'}$, $R^{3'}$ und $R^{4'}$ wie oben definiert sind und

M Wasserstoff oder ein Alkalimetall bedeutet, mit einem Säureanhydrid oder Säurehalogenid einer aliphatischen Mono- oder Dicarbonsäure mit 2-18 C-Atomen, einer heterocyclischen Carbonsäure, die Stickstoff enthält oder einer Aminosäure in einem organischen Lösungsmittel umsetzt.

**Claims** (for the Contracting States : BE, CH, DE, FR, GB, IT, LU, NL, SE)

1. Substituted acetophenones of the formula

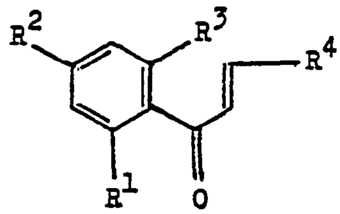

(I)

wherein

$R^1$ represents hydroxy, acyloxy derived from an aliphatic mono- or dicarboxylic acid with 2-18 C-atoms or a heterocyclic carboxylic acid, which contains nitrogen, or aminoacyloxy ;

$R^2$ represents lower alkoxy ;

$R^3$ represents hydrogen or lower alkoxy ; and

$R^4$ represents a phenyl residue, which can be substituted by one or more substituents from the group lower alkyl, lower alkoxy, benzyloxy, allyloxy, alkylthio, dialkylamino, amino, cyano, hydroxy, halogen and alkylenedioxy, a pyridyl, furyl, thienyl or pyrrolyl residue which is optionally substituted by lower alkyl, for the therapeutic treatment of the human or animal body against viruses.

2. The compound 4'-ethoxy-2'-hydroxy-4,6'-dimethoxychalcone for the therapeutic treatment of the human or animal body against viruses.

3. Pharmaceutical preparations, characterized in that they contain a compound of the formula

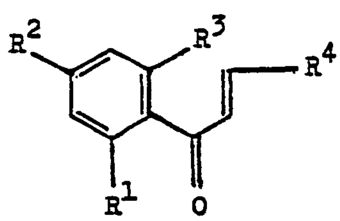

(I)

wherein

$R^1$ represents hydroxy, acyloxy derived from an aliphatic mono- or dicarboxylic acid with 2-18 C-atoms or a heterocyclic carboxylic acid, which contains nitrogen, or aminoacyloxy ;

$R^2$ represents lower alkoxy ;

$R^3$ represents hydrogen or lower alkoxy ; and

$R^4$ represents a phenyl residue, which can be substituted by one or more substituents from the group lower alkyl, lower alkoxy, benzyloxy, allyloxy, alkylthio, dialkylamino, amino, cyano, hydroxy, halogen and alkylenedioxy, a pyridyl, furyl, thienyl or pyrrolyl residue which is optionally substituted by lower alkyl, and an inert carrier material.

4. Pharmaceutical preparations in accordance with claim 3, characterized in that they contain 4'-ethoxy-2'-hydroxy-4,6'-dimethoxychalcone.

5. Substituted acetophenones of the general formula

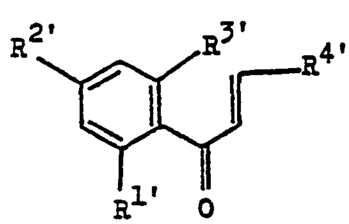

(II)

wherein

$R^{1'}$ represents hydroxy, acyloxy derived from an aliphatic mono- or dicarboxylic acid with 2-18 C-atoms or a heterocyclic carboxylic acid, which contains nitrogen, or aminoacyloxy ;

$R^{2'}$ represents lower alkoxy ;

$R^{3'}$ represents lower alkoxy ; and

$R^{4'}$ represents a phenyl residue, which can be substituted by one or more substituents from the group lower alkyl, lower alkoxy, allyloxy, alkylthio, dialkylamino, amino and cyano, furyl which is substituted by lower alkyl or pyridyl, thienyl or pyrrolyl which is optionally substituted by lower alkyl, with the provisos that when $R^{4'}$ is p-methoxyphenyl, $R^{1'}$ does not represent an acetoxy residue, and that $R^{2'}$ and $R^{3'}$ are not simultaneously methoxy or ethoxy when $R^{1'}$ represents hydroxy and $R^{4'}$ represents methoxyphenyl or dimethoxyphenyl.

6. 4'-Ethoxy-2'-hydroxy-4,6'-dimethoxychalcone.

7. Process for the manufacture of substituted acetophenones of the general formula

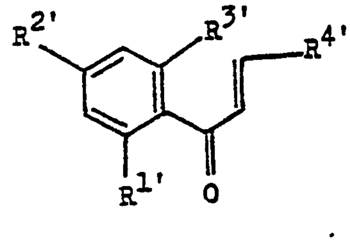

(II)

wherein

$R^{1'}$ represents hydroxy, acyloxy derived from an aliphatic mono- or dicarboxylic acid with 2-18 C-atoms or a heterocyclic carboxylic acid, which contains nitrogen, or aminoacyloxy ;

$R^{2'}$ represents lower alkoxy ;

$R^{3'}$ represents lower alkoxy ; and

$R^{4'}$ represents a phenyl residue, which can be substituted by one or more substituents from the group lower alkyl, lower alkoxy, allyloxy, alkylthio, dialkylamino, amino and cyano, furyl which is substituted by lower alkyl or pyridyl, thienyl or pyrrolyl which is optionally substituted by lower alkyl, with the provisos that when $R^{4'}$ is p-methoxyphenyl, $R^{1'}$ does not represent an acetoxy residue, and that $R^{2'}$ and $R^{3'}$ are not simultaneously methoxy or ethoxy when $R^{1'}$ represents hydroxy and $R^{4'}$ represents methoxyphenyl or dimethoxyphenyl, characterized by

a) for the manufacture of compounds of formula II with $R^{1'}$ = hydroxy, reacting a compound of the formula

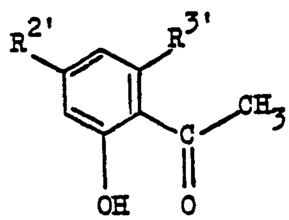

(III)

wherein

$R^{2'}$ and $R^{3'}$ are as defined above, with an aldehyde of the formula

$$R^{4'}\text{—CHO} \qquad \text{(IV)}$$

wherein

$R^{4'}$ is as defined above, in an organic solvent in the presence of a basic catalyst, or

b) for the manufacture of compounds of formula II in which $R^{1'}$ represents an acyloxy residue as defined above or an aminoacyloxy residue, reacting a compound of the formula

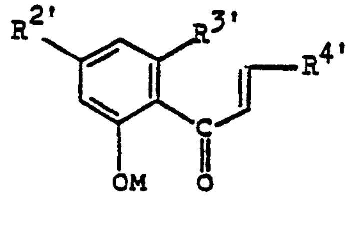

(V)

wherein

$R^{2'}$, $R^{3'}$ and $R^{4'}$ are as defined above and M signifies hydrogen or an alkali metal, with an acid

anhydride or acid halide of an aliphatic mono- or dicarboxylic acid with 2-18 C-atoms, a heterocyclic carboxylic acid, which contains nitrogen, or an amino acid in an organic solvent.

**Claim** (for the Contracting State AT)

1. Process for the manufacture of substituted acetophenones of the general formula

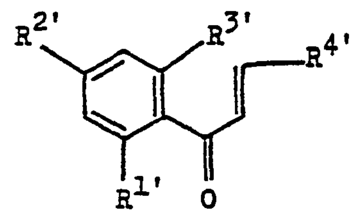

(II)

wherein

$R^{1'}$ represents hydroxy, acyloxy derived from an aliphatic mono- or dicarboxylic acid with 2-18 C-atoms or a heterocyclic carboxylic acid, which contains nitrogen, or aminoacyloxy ;

$R^{2'}$ represents lower alkoxy ;

$R^{3'}$ represents lower alkoxy ; and

$R^{4'}$ represents a phenyl residue, which can be substituted by one or more substituents from the group lower alkyl, lower alkoxy, allyloxy, alkylthio, dialkylamino, amino and cyano, furyl which is substituted by lower alkyl or pyridyl, thienyl or pyrrolyl which is optionally substituted by lower alkyl, with the provisos that when $R^{4'}$ is p-methoxyphenyl, $R^{1'}$ does not represent an acetoxy residue, and that $R^{2'}$ and $R^{3'}$ are not simultaneously methoxy or ethoxy when $R^{1'}$ represents hydroxy and $R^{4'}$ represents methoxyphenyl or dimethoxyphenyl, characterized by

a) for the manufacture of compounds of formula II with $R^{1'}$ = hydroxy, reacting a compound of the formula

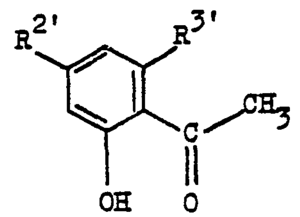

(III)

wherein

$R^{2'}$ and $R^{3'}$ are as defined above, with an aldehyde of the formula

$$R^{4'}\text{—CHO} \qquad \text{(IV)}$$

wherein

$R^{4'}$ is as defined above, in an inert organic solvent in the presence of a basic catalyst, or

b) for the manufacture of compounds of formula II in which $R^{1'}$ represents an acyloxy residue as defined above or an aminoacyloxy residue, reacting a compound of the formula

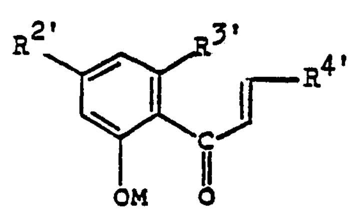

(V)

wherein

$R^{2'}$, $R^{3'}$ and $R^{4'}$ are as defined above and M signifies hydrogen or an alkali metal, with an acid anhydride or acid halide of an aliphatic mono- or dicarboxylic acid with 2-18 C-atoms, a heterocyclic carboxylic acid, which contains nitrogen, or an amino acid in an organic solvent.

**Revendications** (pour les Etats contractants : BE, CH, DE, FR, GB, IT, LU, NL, SE)

1. Acétophénones substituées de formule :

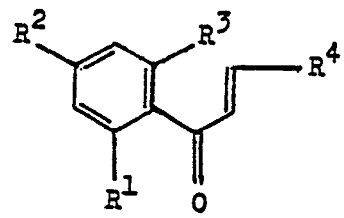

(I)

où

$R^1$ représente un hydroxy, un acyloxy dérivé d'un acide mono- ou dicarboxylique en $C_2$ à $C_{18}$ ou d'un acide carboxylique hétérocyclique qui contient de l'azote ; ou un aminoacyloxy ;

$R^2$ représente un alcoxy inférieur,

$R^3$ un hydrogène ou un alcoxy inférieur ; et

$R^4$ un radical phényle, qui peut être substitué par un ou plusieurs substituants du groupe alcoyle inférieur, alcoxy inférieur, benzyloxy, allyloxy, alcoylthio, dialcoylamino, amino, cyano, hydroxy, halogène et alcoylènedioxy, un radical pyridyle, furyle, thiényle ou pyrrolyle éventuellement substitué par un alcoyle inférieur, pour le traitement thérapeutique du corps humain ou animal contre les virus.

2. Le composé 4'-éthoxy-2'-hydroxy-4,6'-diméthoxychalcone aux fins de traitement thérapeutique du corps humain ou animal contre les virus.

3. Préparations pharmaceutiques, caractérisées en ce qu'elles contiennent un composé de formule :

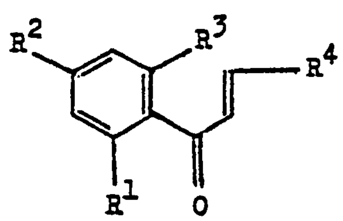

(I)

où

$R^1$ représente un hydroxy, un acyloxy dérivé d'un acide mono- ou dicarboxylique en $C_2$ à $C_{18}$ ou d'un acide carboxylique hétérocyclique qui contient de l'azote ; ou un aminoacyloxy ;

$R^2$ représente un alcoxy inférieur ;

$R^3$ un hydrogène ou un alcoxy inférieur ; et

$R^4$ un radical phényle, qui peut être substitué par un ou plusieurs substituants du groupe alcoyle inférieur, alcoxy inférieur, benzyloxy, allyloxy, alcoylthio, dialcoylamino, amino, cyano, hydroxy, halogène et alcoylènedioxy, un radical pyridyle, furyle, thiényle ou pyrrolyle éventuellement substitué par un alcoyle inférieur, et un support.

4. Préparations pharmaceutiques selon la revendication 3, caractérisées en ce qu'elles contiennent la 4'-éthoxy-2'-hydroxy-4,6'-diméthoxychalcone.

5. Acétophénones substituées de formule générale :

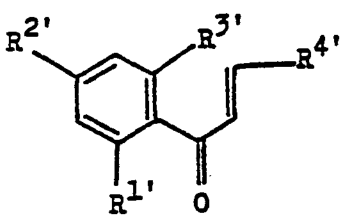

(II)

où

$R^{1'}$ représente un hydroxy, un acyloxy dérivé d'un acide mono- ou dicarboxylique aliphatique en $C_2$ à $C_{18}$ ou d'un acide carboxylique qui contient de l'azote ; ou un aminoacyloxy ;

$R^{2'}$ représente un alcoxy inférieur ;

$R^{3'}$ un alcoxy inférieur ; et

$R^{4'}$ un radical phényle, qui peut être substitué par un ou plusieurs substituants du groupe alcoyle inférieur, alcoxy inférieur, allyloxy, alcoylthio, dialcoylamino, amino et cyano, un furyle substitué par un alcoyle inférieur ou un pyridyle, thiényle, ou pyrrolyle éventuellement substitué par un alcoyle inférieur,

avec comme condition limitative que lorsque $R^{4'}$ est un p-méthoxyphényle, $R^{1'}$ ne représente pas un radical acétoxy, et que $R^{2'}$ et $R^{3'}$ ne représentent pas simultanément un méthoxy ou un éthoxy lorsque $R^{1'}$ représente un hydroxy et $R^{4'}$ un méthoxyphényle ou un diméthoxyphényle.

6. 4'-éthoxy-2'-hydroxy-4,6'-diméthoxychalcone.

7. Procédé de préparation d'acétophénones substituées de formule générale :

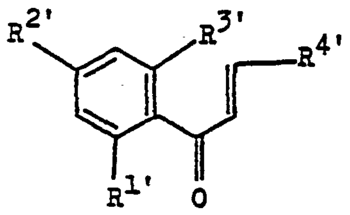

(II)

où

$R^{1'}$ représente un hydroxy, un acyloxy dérivé d'un acide mono- ou dicarboxylique aliphatique en $C_2$ à $C_{18}$ ou d'un acide carboxylique hétérocyclique qui contient de l'azote ; ou un aminoacyloxy ;

$R^{2'}$ représente un alcoxy inférieur ;

$R^{3'}$ un alcoxy inférieur ; et

$R^{4'}$ un radical phényle, qui peut être substitué par un ou plusieurs substituants du groupe alcoyle inférieur, alcoxy inférieur, allyloxy, alcoylthio, dialcoylamino, amino et cyano, un furyle substitué par un alcoyle inférieur ou un pyridyle, thiényle ou pyrrolyle éventuellement substitué par un alcoyle inférieur, avec les précisions limitatives que lorsque $R^{4'}$ est un p-méthoxyphényle, $R^{1'}$ ne représente pas un radical acétoxy, et que $R^{2'}$ et $R^{3'}$ ne sont pas simultanément un méthoxy ou un éthoxy lorsque $R^{1'}$ représente un hydroxy et $R^{4'}$ un méthoxyphényle ou un diméthoxyphényle, caractérisé en ce que :

a) pour préparer des composés de formule (II) avec $R^{1'}$ = hydroxy, on fait réagir un composé de formule :

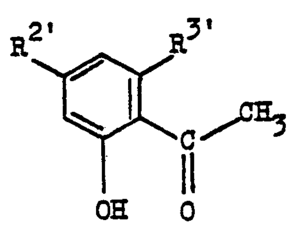

(III)

où

$R^{2'}$ et $R^{3'}$ sont tels que définis ci-dessus, avec un aldéhyde de formule :

$$R^{4'}\text{—CHO} \qquad \text{(IV)}$$

où

$R^{4'}$ est tel que défini ci-dessus, dans un solvant organique en présence d'un catalyseur basique, ou

b) pour préparer des composés de formule (II) où $R^{1'}$ représente un radical acyloxy ou un radical aminoacyloxy tel que défini ci-dessus, on fait réagir un composé de formule :

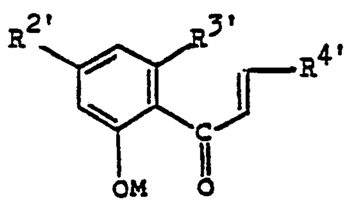

(V)

où

$R^{2'}$, $R^{3'}$ et $R^{4'}$ sont tels que définis ci-dessus et où M représente un hydrogène ou un métal alcalin, avec un anhydride d'acide ou un halogénure d'acide d'un acide mono- ou dicarboxylique aliphatique en $C_2$ à $C_{18}$, d'un acide carboxylique hétérocyclique qui contient de l'azote, ou un acide aminé dans un solvant organique.

**Revendication** (pour l'Etat contractant AT)

1. Procédé de préparation d'acétophénones substituées de formule générale :

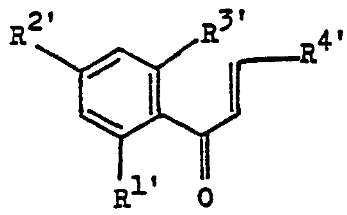

(II)

où

$R^{1'}$ représente un hydroxy, un acyloxy dérivé d'un acide mono- ou dicarboxylique aliphatique en $C_2$ à $C_{18}$ ou d'un acide carboxylique hétérocyclique qui contient de l'azote ; ou un aminoacyloxy ;

$R^{2'}$ représente un alcoxy inférieur ;

$R^{3'}$ un alcoxy inférieur, et

$R^{4'}$ un radical phényle, qui peut être substitué par un ou plusieurs substituants du groupe alcoyle inférieur, alcoxy inférieur, allyloxy, alcoylthio, dialcoylamino, amino et cyano, un furyle substitué par un alcoyle inférieur, ou un pyridyle, un thiényle ou un pyrrolyle éventuellement substitué par un alcoyle inférieur, avec les précisions limitatives que lorsque $R^{4'}$ est un p-méthoxy-phényle, $R^{1'}$ ne représente pas un radical acétoxy, et que $R^{2'}$ et $R^{3'}$ ne représentent pas simultanément un méthoxy ou un éthoxy lorsque $R^{1'}$ représente un hydroxy et $R^{4'}$ un méthoxyphényle ou un diméthoxyphényle, caractérisé en ce que :

a) pour préparer des composés de formule (II) où $R^{1'}$ = hydroxy, on fait réagir un composé de formule :

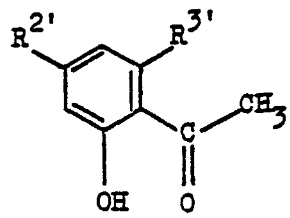

(III)

où

$R^{2'}$ et $R^{3'}$ sont tels que définis ci-dessus, avec un aldéhyde de formule :

$$R^{4'}—CHO \qquad (IV)$$

où

$R^{4'}$ est tel que défini ci-dessus, dans un solvant organique, en présence d'un catalyseur basique, ou

b) pour préparer des composés de formule (II) où $R^{1'}$ représente un radical acyloxy ou un radical aminoacyloxy tel que défini ci-dessus, on fait réagir un composé de formule :

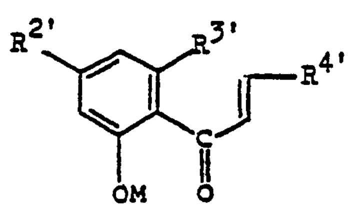

(V)

où

$R^{2'}$, $R^{3'}$ et $R^{4'}$ sont tels que définis ci-dessus et M représente un hydrogène ou un métal alcalin, avec un anhydride d'acide ou un halogénure d'acide d'un acide mono- ou dicarboxylique aliphatique en $C_2$ à $C_{18}$, d'un acide carboxylique hétérocyclique qui contient de l'azote, ou d'un acide aminé dans un solvant organique.

Fig. 1: Hemmung der Vermehrung von Rhinovirus durch 2'-Hydroxy-4,4',6'-trimethoxychalkon

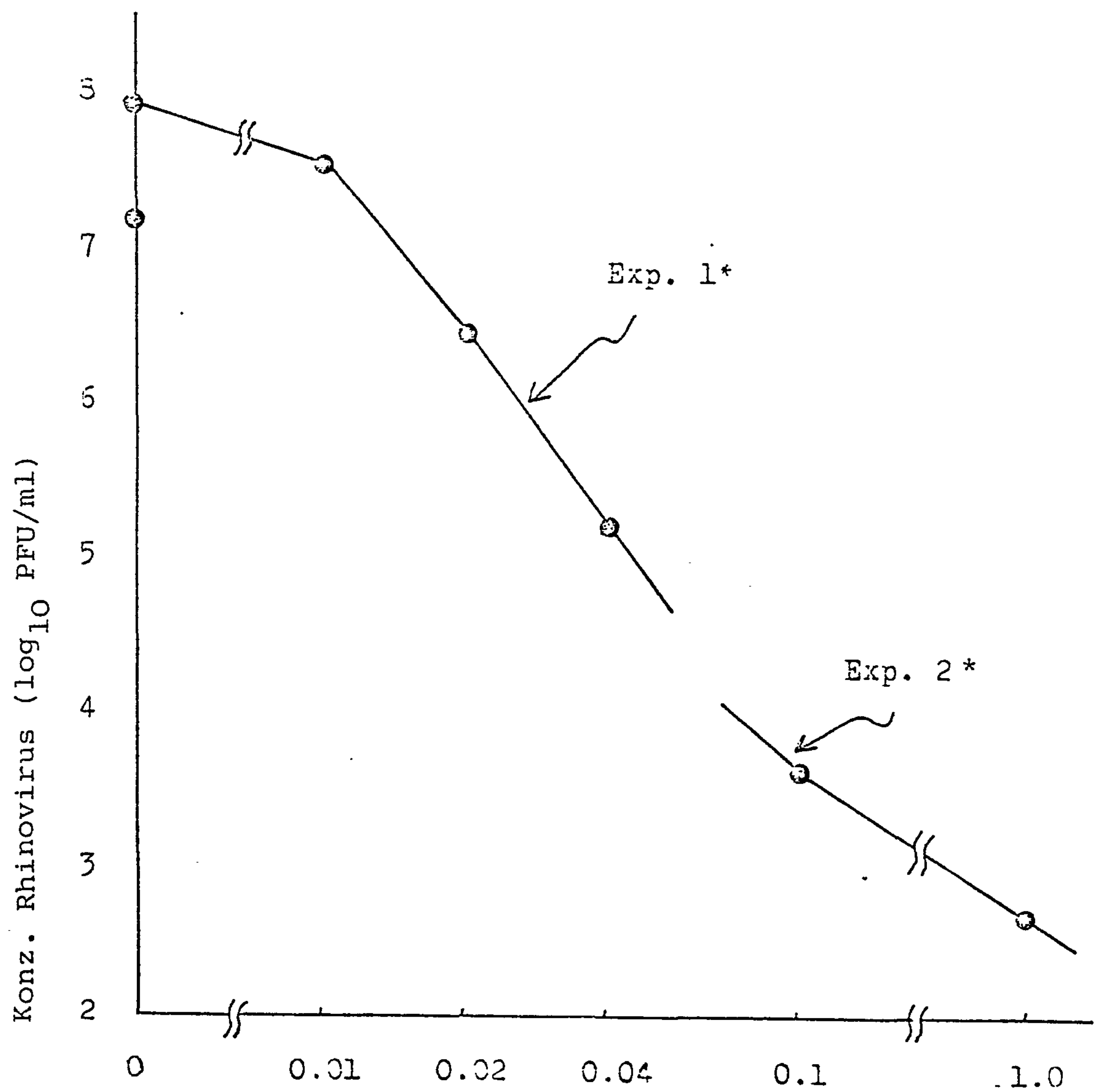


zugesetztes 2'-Hydroxy-4,4',6'-trimethoxychalkon (µg/ml)

*) Ergebnisse aus zwei Versuchen

PFU = Plaque-bildende Einheiten

Fig. 2:

Hemmung der Vermehrung von Rhinovirus Typ 21
durch 4'-Ethoxy-2'-hydroxy-4,6'-dimethoxychalkon